# EUROPEAN PATENT APPLICATION

(11) **EP 4 394 790 A1**
(43) Date of publication of application: **03.07.2024**
(21) Application number: 23217796.4
(22) Date of filing: 18.12.2023
(51) Int. Cl.: G16H 20/60

(54) **METHOD AND APPARATUS FOR MOBILE HEALTHCARE SERVICE USING DIET MANAGEMENT**

(30) Priority: 30.12.2022 KR 20220190731; 23.11.2023 KR 20230164324
(71) Applicant: Nuvi Labs Co., Ltd., Seoul 06173 (KR)
(72) Inventor: KIM, Dae Hoon, 06173 Seoul (KR); JUN, Yeo Jung, 06173 Seoul (KR)
(74) Representative: Habermann, Hruschka & Schnabel

(57) **Abstract**

Disclosed is a method and apparatus for mobile healthcare service using diet management. The mobile healthcare service includes: when any food is detected on a photoshoot screen upon execution of a camera by a user, displaying a detected food area; when the user captures a photo of the photoshoot screen displaying the food area, cropping the food area from the captured photo; when a consumed food is selected by the user in the cropped food area, recording the selected consumed food as dietary data; and providing a mobile healthcare service using diet management by comparing the dietary data with a preset diet management goal.

## Description

### CROSS-REFERENCE TO RELATED APPLICATIONS

This application claims priority to Korean Patent Application No. 10-2022-0190731 filed on 30 Dec, 2022 and Korean Patent Application No. 10-2023-0164324 filed on 23 Nov, 2023 in Korea, the entire contents of which are hereby incorporated by reference in their entirety.

### TECHNICAL FIELD

The present disclosure relates to a method and apparatus for mobile healthcare service using diet management.

### BACKGROUND

Recently, with the increasing focus on well-being life, there has been a growing interest among people in health. Consequently, numerous wellness foods aimed at maintaining health have emerged in response to this heightened interest. Moreover, there is a rising demand from people to maintain their health through dietary control, even without consuming wellness foods.

Particularly, individuals such as patients with conditions like diabetes, hypertension, gestational diabetes, or athletes requiring weight control, necessitate consistent dietary management.

Consequently, various dietary management programs have been developed to record and assess users' dietary intake. Most of these programs primarily focus on making it easy for users to input and record their consumed diets.

However, even with the ease of inputting diets, recording itself can be challenging. In situations where recording immediately after meals is not feasible or when users simply forget to record, relying on memory from past instances can lead to difficulties and reduced accuracy in documenting the consumed diet. Moreover, the task becomes more challenging when users consume a wide variety of foods, such as Korean cuisine or multi-course meals, as recalling and documenting the diet consumed after a significant period can be troublesome.

Therefore, there is a demand for technologies that allow users to conveniently input their diets. Additionally, technologies enabling users to accurately remember, record, and manage their diets even after an extended period following meals or after consuming diverse foods are highly sought after.

### SUMMARY

The present disclosure provides a method and apparatus for a mobile healthcare service using diet management. The mobile healthcare service includes detecting a food area on a photoshoot screen, cropping the detected food area, recording the food area into a diet, and comparing the food area with a preset diet management goal, thereby enabling users to conveniently utilize the mobile healthcare service using diet management.

However, the problem to be solved by the present disclosure is not limited to this, and may be expanded in various environments without departing from the spirit and scope of the present disclosure.

In one aspect of the present disclosure, there is provided a mobile healthcare service method performed by a mobile healthcare service apparatus, the method including: when any food is detected on a photoshoot screen upon execution of a camera by a user, displaying a detected food area; when the user captures a photo of the photoshoot screen displaying the food area, cropping the food area from the captured photo; when a consumed food is selected by the user in the cropped food area, recording the selected consumed food as dietary data; and providing a mobile healthcare service using diet management by comparing the dietary data with a preset diet management goal.

In displaying the food area, a distinct food area for each type of food located in the photoshoot screen may be displayed.

In recording the selected consumed food as the dietary data, the dietary data may include metadata included in the selected consumed food and the captured photo.

The method may further include generating an avatar in a virtual space based on the user's body information, creating virtual clothing, dressing the avatar with the generated virtual clothing, and adjusting a clothing fit of the avatar wearing the virtual clothing based on the recorded dietary data and the preset dietary management goal.

In recording the selected consumed food as the dietary data, a quantity of the selected consumed food may be calculated using restaurant menu data corresponding to the user's location information, and the calculated quantity may be recorded as dietary data.

In providing the mobile healthcare service, a recommended meal order for consuming the detected food may be provided to the user based on the preset diet management goal.

In providing the mobile healthcare service, a food intake warning may be issued for any detected food that doesn't align with the preset dietary management goal.

The method may further include searching for a Doppelganger user exceeding a preset similarity threshold with the recorded dietary data and the preset dietary management goal, and providing dietary data and diet preference information of the found doppelganger user.

The method may further include providing at least one recommendation information suitable for the user based on the found doppelganger user's dietary data and preference information.

In another aspect of the present disclosure, there is provided a mobile healthcare service apparatus using diet management, the apparatus including: a camera; a display; a memory configured to store one or more programs; and a processor configured to execute the one or more stored programs. The processor is further configured to: when any food is detected on a photoshoot screen of the display upon execution of the camera by a user, display a detected food area; when the user captures a photo of the photoshoot screen showing the food area, crop the food area from the captured photo; when a consumed food is selected by the user in the cropped food area, recording the selected consumed food as dietary data; and provide a mobile healthcare service using diet management by comparing the recorded dietary data with a preset diet management goal.

The processor may be further configured to display a distinct food areas for each type of food located in the photoshoot screen of the display.

The processor may be further configured to record dietary data that comprises metadata included in the selected consumed food and the captured photo.

The processor may be further configured to generate an avatar in a virtual space based on the user's body information, create virtual clothing, dress the avatar with the generated virtual clothing, and adjust a clothing fit of the avatar dressed with the virtual clothing based on the recorded dietary data and the preset dietary management goal.

The processor may be further configured to calculate a quantity of the selected consumed food using restaurant menu data corresponding to the user's location information and record the calculated quantity as dietary data.

The processor may be further configured to provide the user with a recommended meal order for consuming the detected food based on the preset diet management goal.

The processor may be further configured to issue a food intake warning for any detected food that doesn't align with the preset dietary management goal.

The processor may be further configured to search for a doppelgänger user exceeding a preset similarity threshold with the recorded dietary data and the preset dietary management goal, and provide dietary data and diet preference information of the found doppelgänger user.

The processor may be further configured to provide at least one recommendation information suitable for the user based on the found doppelganger user's dietary data and preference information.

The disclosed technology may have the following effects. However, it is not meant to imply that a particular embodiment should include all or only these effects. Therefore, the scope of the disclosed technology should not be understood as being limited thereto.

In embodiments of the present disclosure, there is provided a mobile healthcare service including detecting a food area on a photoshoot screen, cropping the detected food area, recording the food area into a diet, and comparing the food area with a preset diet management goal, thereby enabling users to conveniently utilize the mobile healthcare service using diet management.

### BRIEF DESCRIPTION OF THE DRAWINGS

FIG.1 is a diagram illustrating a configuration of a mobile healthcare service system using diet management according to an embodiment of the present disclosure.
FIG.2 is a flowchart illustrating an operation of recording and analyzing a diet based on a food photo by a mobile healthcare service apparatus according to an embodiment of the present disclosure.
FIG.3 is a flowchart illustrating an operation of displaying a food area and recording a dietary by a mobile healthcare service apparatus according to an embodiment of the present disclosure.
FIG.4 is a flowchart illustrating an operation for a diet recording challenge and goal attainment analysis by a mobile healthcare service apparatus according to an embodiment of the present disclosure.
FIG.5 is a flowchart illustrating an operation of attaining a clothing fit goal using an avatar in a virtual space by a mobile healthcare service apparatus according to an embodiment of the present disclosure.
FIGS. 6 and 7 are diagrams showing examples of diet management and analysis by a mobile healthcare service apparatus according to an embodiment of the present disclosure.
FIGS. 8 and 9 are diagrams showing an example of requesting dietary analysis by a mobile healthcare service apparatus according to an embodiment of the present disclosure.
FIGS. 10 and 11 are diagrams showing an example of setting a target calorie and nutrient by a mobile healthcare service apparatus according to an embodiment of the present disclosure.
FIG. 12 is a diagram showing an example of a diet recording challenge operation by a mobile healthcare service apparatus according to an embodiment of the present disclosure.
FIGS. 13 and 14 are diagrams showing an example of a goal attainment report provided by a mobile healthcare service apparatus according to an embodiment of the present disclosure.
FIGS. 15 and 16 are diagrams showing an example of a food photo search operation in an album by a mobile healthcare service apparatus according to an embodiment of the present disclosure.
FIGS. 17 and 18 are diagrams showing an example of displaying a calorie flow and expected calorie intake by a mobile healthcare service apparatus according to an embodiment of the present disclosure.
FIGS. 19 and 20 are diagrams illustrating an example of quickly recording a diet with favorite foods by a mobile healthcare service apparatus according to an embodiment of the present disclosure.
FIGS. 21 and 22 are diagrams showing an example of a simple diet recording operation using photos by a mobile healthcare service apparatus according to an embodiment of the present disclosure.
FIGS. 23 and 24 are diagrams showing an example of searching for and recording a diet by a mobile healthcare service apparatus according to an embodiment of the present disclosure.
FIGS. 25 and 26 are diagrams showing an example of an operation of requesting nutrient analysis of an unfound item by a mobile healthcare service apparatus according to an embodiment of the present disclosure.
FIGS. 27 and 28 are diagrams showing examples of a result of food photo analysis by a mobile healthcare service apparatus according to an embodiment of the present disclosure.
FIGS. 29 and 30 are diagrams showing an example of an operation of editing favorite foods by a mobile healthcare service apparatus according to an embodiment of the present disclosure.
FIG.31 is a diagram showing an example of menu analysis settings by a mobile healthcare service apparatus according to an embodiment of the present disclosure.
FIG.32 is a diagram showing an example of a camera album by a mobile healthcare service apparatus according to an embodiment of the present disclosure.
FIG.33 is a diagram showing an example of a camera AR operation by a mobile healthcare service apparatus according to an embodiment of the present disclosure.
FIG.34 is a diagram showing an example of individual and team competition actions for a diet challenge by a mobile healthcare service apparatus according to an embodiment of the present disclosure.
FIG.35 is a diagram showing an example of an analysis S curve by a mobile healthcare service apparatus according to an embodiment of the present disclosure.
FIG.36 is a diagram showing an example of an eating habits analyzing operation by a mobile healthcare service apparatus according to an embodiment of the present disclosure.
FIG.37 is a diagram showing an example of a vegetarianism authentication operation by a mobile healthcare service apparatus according to an embodiment of the present disclosure.
FIG.38 is a diagram showing an example of a net-zero authentication operation by a mobile healthcare service apparatus according to an embodiment of the present disclosure.
FIGS. 39 and 40 are diagrams showing examples of open chat and friend chat operations by a mobile healthcare service apparatus according to an embodiment of the present disclosure.
FIG.41 is a diagram showing an example of food MBTI by a mobile healthcare service apparatus according to an embodiment of the present disclosure.
FIG.42 is a diagram showing an example of food cash by a mobile healthcare service apparatus according to an embodiment of the present disclosure.
FIG.43 is a diagram showing an example of a personal challenge and group challenge operation by a mobile healthcare service apparatus according to an embodiment of the present disclosure.
FIG.44 is a diagram showing an example of a mentoring and goal attainment mission guide operation by a mobile healthcare service apparatus according to an embodiment of the present disclosure.

### DETAILED DESCRIPTION

The present disclosure may allow various kinds of change or modification and various changes in form, and specific exemplary embodiments will be illustrated in drawings and described in detail in the specification. However, it should be understood that the specific exemplary embodiments do not limit the present disclosure to a specific disclosing form but include every modified, equivalent, or replaced one within the spirit and technical scope of the present disclosure. In describing the present disclosure, if it is determined that a detailed description of known functions and components associated with the present disclosure unnecessarily obscure the gist of the present disclosure, the detailed description thereof will be omitted.

Although terms, such as 'first' and 'second', can be used to describe various components, the components cannot be limited by the terms. The terms may be used to distinguish a certain component from another component.

The terms and expressions used in the present disclosure are used only for the purpose of illustrating particular embodiments, and are not intended to limit the present disclosure. Although general terms as currently widely used as possible are selected as the terms used in the present disclosure while taking functions in the present disclosure into account, they may vary according to an intention of those of ordinary skill in the art, judicial precedents, or the appearance of new technology. In addition, in specific cases, terms intentionally selected by the applicant may be used, and in this case, the meaning of the terms will be disclosed in corresponding description of the disclosure. Accordingly, the terms used in the present disclosure should be defined not by simple names of the terms but by the meaning of the terms and the content over the present disclosure.

An expression in the singular includes an expression in the plural unless they are clearly different from each other in context. It should be noted that the terms "include" or "have" as used in the present disclosure are intended to denote the existence of any features, numerical values, steps, operations, constituent elements, parts, and combinations thereof described in the specification, but are not intended to preliminarily exclude the possibility of existence or addition of any one or more other features, numerical values, steps, operations, constituent elements, parts, and combinations thereof.

Hereinafter, the present disclosure will now be described more fully with reference to the accompanying drawings, in which exemplary embodiments are shown. Like reference numerals in the drawings denote like elements, and thus their repetitive description will be omitted.

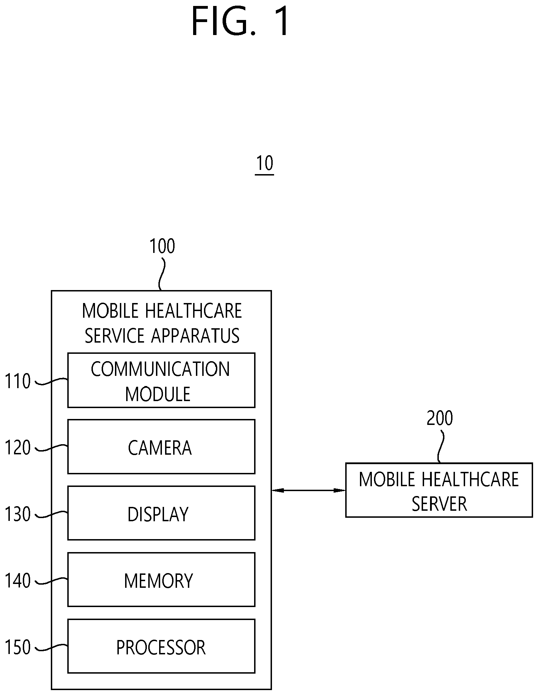

FIG.1 is a diagram illustrating a configuration of a mobile healthcare service system using diet management according to an embodiment of the present disclosure.

As shown in FIG. 1, a mobile healthcare service system 10 using diet management according to an embodiment of the present disclosure includes a mobile healthcare service apparatus 100 and a mobile healthcare service server 200. Here, the mobile healthcare service apparatus 100 may include a communication module 110, a camera 120, a display 130, a memory 140, and a processor 150. However, all of the illustrated components are not essential. The mobile healthcare service system 10 using diet management may be implemented using more or less components than those illustrated in the drawings.

Hereinafter, a specific configuration and operation of each component of the mobile healthcare service system 10 using diet management in FIG. 1 will be described.

The mobile healthcare service apparatus 100 may provide a mobile healthcare service using diet management by displaying a detected food area in a photoshoot screen, cropping the food area from a captured photo, recording the food area into a diet, and comparing the food area with a preset diet management goal. This allows users to conveniently access the mobile healthcare service through diet management. In another example, when a user takes a photo of food with a food area displayed, the mobile healthcare service apparatus 100 automatically records the food as a diet, analyzes a corresponding dietary record, and provides a mobile healthcare service, thereby enhancing mobile convenience for diet management.

The mobile healthcare server 200 may record the user's diet and analyze a dietary record in conjunction with the mobile healthcare service apparatus 100. The mobile healthcare server 200 may process a request for dietary record analysis that cannot be performed by the mobile healthcare service apparatus 100 or a request for analyzing a menu item that cannot be searched, and subsequently provide an analysis result to the mobile healthcare service apparatus 100.

Hereinafter, a specific configuration and operation of each component of the mobile healthcare service apparatus 100 using diet management in FIG. 1 will be described.

The communication module 110 may include one or more modules that enable communication between the mobile healthcare service apparatus 100 and a wireless communication system, or between the mobile healthcare service apparatus 100 and the mobile healthcare server 200. In addition, the communication module 110 may include one or more modules that connect the mobile healthcare service apparatus 100 to one or more networks.

The camera 120 may capture an image or video containing food based on a user's operation. Here, the camera 120 may capture an image or video of food before or after a meal based on the user's operation. The camera 120 may include a single camera, a plurality of cameras, a single image sensor, or a plurality of image sensors. The camera 120 may include at least one of at least one 2D camera, at least one 3D camera, at least one stereo camera, and at least one image sensor.

The display 130 may form a layered structure or is integrally formed with a touch sensor, thereby implementing a touch screen. The touch screen may provide an input interface between the mobile healthcare service apparatus 100 and a user, and an output interface between the mobile healthcare service apparatus 100 and the user.

The memory 140 may store data supporting various functions of the mobile healthcare service apparatus 100. The memory 140 may store one or more programs running on the mobile healthcare service apparatus 100, a plurality of application programs or applications, and data and commands for operation of the mobile healthcare service apparatus 100. At least some of these applications may be downloaded from an external server 200 through wireless communication. In addition, at least some of these applications may be designed for basic functions of the mobile healthcare service apparatus 100. Meanwhile, each application program may be stored in the memory 140 and installed on the mobile healthcare service apparatus 100 to perform the operation (or function) of the mobile healthcare service apparatus 100 under the control of the processor 150.

In addition to operations related to the application program, the processor 150 may generally control the overall operation of the mobile healthcare service apparatus 100. The processor 150 may provide or process appropriate mobile healthcare service information or functions to the user by processing signals, data, information, etc. input or output through the components discussed above or by running an application program stored in the memory 140.

According to embodiments, when any food is detected on the photoshoot screen upon execution of the camera by a user, the processor 150 may display a detected food area on the display 130. Then, when the user captures a photo of the photoshoot screen where the food area is displayed, the processor 150 may crop the food area from the captured photo, and when food consumed by the user is selected in the cropped food area, the processor 150 may record the selected food as at least one diet and compare a recorded dietary record with a preset diet management goal. In this manner, a mobile healthcare service using diet management may be provided.

According to embodiments, the processor 150 may display a distinct food area for each type of food located in the photoshoot screen of the display 130.

According to embodiments, the processor 150 may record dietary data, including selected consumed food and metadata contained in photos taken through the camera 120.

According to embodiments, the processor 150 may generate an avatar tailored to the user's body information in a virtual space, generate virtual clothing, dress the avatar with the virtual clothing, and adjust the clothing fit of the avatar dressed with the virtual clothing based on recorded dietary data and a preset dietary management goal.

According to embodiments, the processor 150 may calculate a consumed quantity of the selected food using restaurant menu data corresponding to the user's location information and record the calculated quantity as dietary data.

As an example, the processor 150 may retrieve menu data from a restaurants where the user is dining, using a location positioning device (such as GPS, beacons, Bluetooth, Wi-Fi, indoor tracking devices, etc.), and accurately calculate a quantity of the food consumed by the user based on the retrieved menu data. For example, even if multiple restaurants offer the same food item, the quantity and nutritional content of that item may vary across the restaurants due to differences in ingredients or cooking methods. Here, the processor 150 may obtain menu data at the location of a restaurant where the user is dining, using a location positioning device. Therefore, the processor 150 may accurately calculate the quantity of consumed food and nutrients included in the food based on the acquired menu data, and record the calculated quantity as dietary data. At this point, the processor 150 may accurately identify even newly introduced menu items that haven't been previously learned, using the menu data from the restaurant where the user is dining.

According to embodiments, the processor 150 may provide the user with a recommended meal sequence for intaking detected food items based on a preset dietary management goal.

According to embodiments, the processor 150 may issue a food intake warning for any detected food item that does not align with the preset dietary management goal.

According to embodiments, the processor 150 may search for a doppelgänger user exceeding a preset similarity threshold with recorded dietary data and the preset dietary management goal, and then provide dietary data and at least one preference information of the found doppelgänger user.

According to embodiments, the processor 150 may provide at least one recommendation information tailored to the user based on the found doppelganger user's dietary data and at least one preference information.

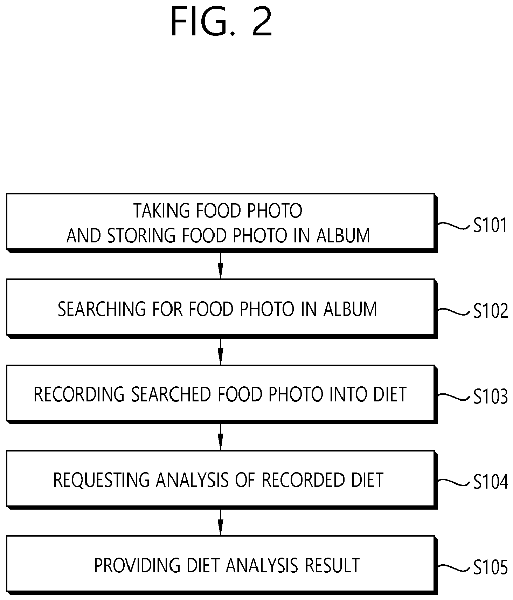

FIG.2 is a flowchart illustrating an operation of recording and analyzing a diet based on a food photo by a mobile healthcare service apparatus according to an embodiment of the present disclosure.

In operation S 101, the mobile healthcare service apparatus 100 obtains a food photo upon the user's photographing food, and stores the obtained food photo in an album. This food photo may be selected as a dietary record containing foods consumed by the user.

In operation S 102, the mobile healthcare service apparatus 100 may search for a food photo in real time or periodically from an album in which food photos are previously stored. The album contains not only food photos but also general pictures of the user. When the user captures and saves a food photo, the mobile healthcare service apparatus 100 may search for the food photo among photos stored in the album.

In operation S 103, the mobile healthcare service apparatus 100 may record the found food photo into a diet. Here, the mobile healthcare service apparatus 100 may allow the user to manually select a food photo, or may automatically record a food photo where any food has been detected among the photos into the user's diet. At this point, metadata contained in the food photo (e.g., the date of food capture, time, location, etc.) may also be added to a dietary record. Alternatively, the mobile healthcare service apparatus 100 may analyze the user's food photo to determine whether it is a pre-meal or post-meal photo and then record the photo into a diet.

In operation S104, the mobile healthcare service apparatus 100 may receive a request from the user for analyzing the recorded dietary entry. Here, the mobile healthcare service apparatus 100 may analyze the dietary entry, which includes saved food photos for a predetermined period of time (such as a day, a week, a month, etc.), without a specific request for dietary analysis from the user.

In operation S104, the mobile healthcare service apparatus 100 may provide a dietary analysis result to the user. In addition, the mobile healthcare service apparatus 100 may provide various mobile healthcare services using the dietary analysis result.

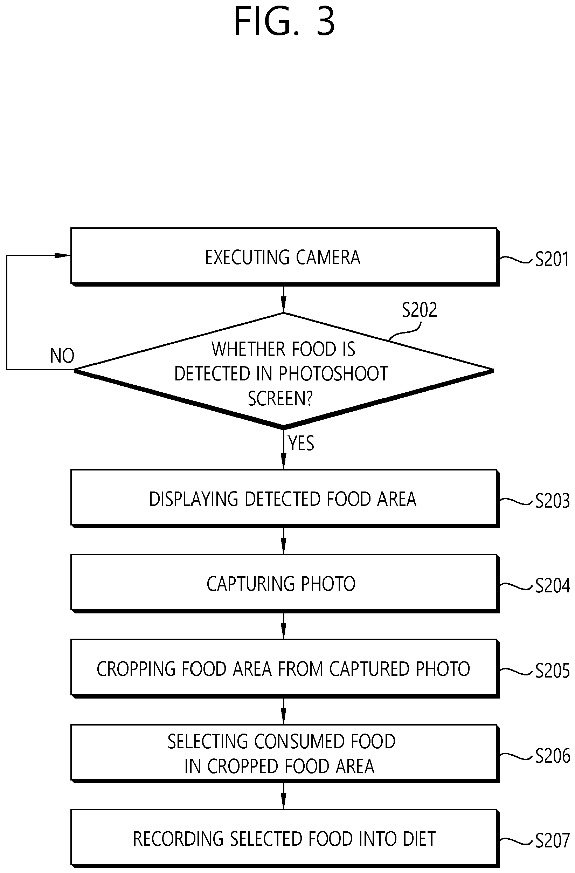

FIG.3 is a flowchart illustrating an operation of displaying a food area and recording a dietary by a mobile healthcare service apparatus according to an embodiment of the present disclosure.

In operation S201, a mobile healthcare service apparatus 100 displays a photoshoot screen on a display when a user executes the camera.

In operation S202, the mobile healthcare service apparatus 100 verifies whether any food is detected in the photoshoot screen when the user executes the camera.

In operation S203, when any food is detected on the photoshoot screen upon the user's executing the camera, the mobile healthcare service apparatus 100 displays a detected food area. Here, the mobile healthcare service apparatus 100 may display a distinct food area for each of at least one food located on the photoshoot screen. In another example, the mobile healthcare service apparatus 100 may display a distinct food area for each type of food located on the photoshoot screen. In yet another example, the mobile healthcare service apparatus 100 may display a distinct food area for each of at least one food category (e.g., rice, side dish, soup, etc.) located on the photoshoot screen. In yet another example, the mobile healthcare service apparatus 100 may display a distinct food area for each of at least one food located on the photoshoot screen based on preset food area information, taking into account the user's individual characteristics. If any food is not detected in real time on the photoshoot screen, the mobile healthcare service apparatus 100 continues to perform operation S201 of displaying the photoshoot screen by executing the camera.

In operation S204, the mobile healthcare service apparatus 100 verifies whether the user captures a photo of the photoshoot screen displaying the food area, and stores the captured photo.

In operation S205, when the user captures a photo of the photoshoot screen displaying the food area, the mobile healthcare service apparatus 100 crops the food area from the captured photo.

In operation S206, the mobile healthcare service apparatus 100 receives a user's selection of food from the cropped food area.

In operation S207, upon receiving the user's selection of food from the cropped food area, the mobile healthcare service apparatus 100 may record the selected food as at least one dietary entry. Thereafter, the mobile healthcare service apparatus 100 may provide a mobile healthcare service using diet management by comparing at least one recorded dietary record with a preset diet management goal.

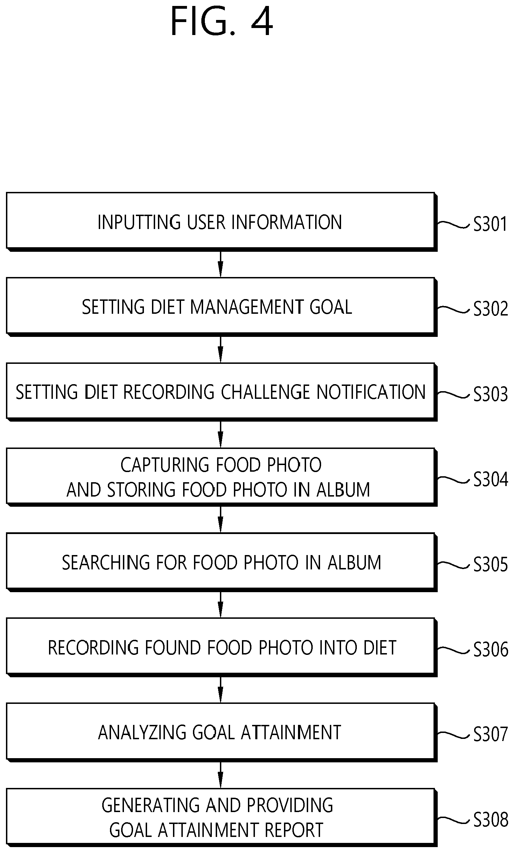

FIG.4 is a flowchart illustrating an operation for a diet recording challenge and goal attainment analysis by a mobile healthcare service apparatus according to an embodiment of the present disclosure.

In operation S301, a mobile healthcare service apparatus 100 receives user information from a user.

In operation S302, the mobile healthcare service apparatus 100 sets a diet management goal based on the user's input.

In operation S303, the mobile healthcare service apparatus 100 sets a notification for a diet recording challenge in accordance with the dietary management goal.

In operation S304, the mobile healthcare service apparatus 100 obtains a food photo through the user's taking a photo of food, and stores the obtained food photo in an album.

In operation S305, the mobile healthcare service apparatus 100 may search for a food photo in real time or periodically from the album in which food photos are previously stored.

In operation S306, the mobile healthcare service apparatus 100 may record the searched food photo into a diet.

In operation S307, the mobile healthcare service apparatus 100 may analyze whether the dietary management goal has been attained by comparing an analysis result of the recorded dietary entry with the preset dietary management goal.

In operation S308, the mobile healthcare service apparatus 100 may generate an attainment report for the dietary management goal based on the analysis result of the dietary management goal, and subsequently provide the generated attainment report to the user.

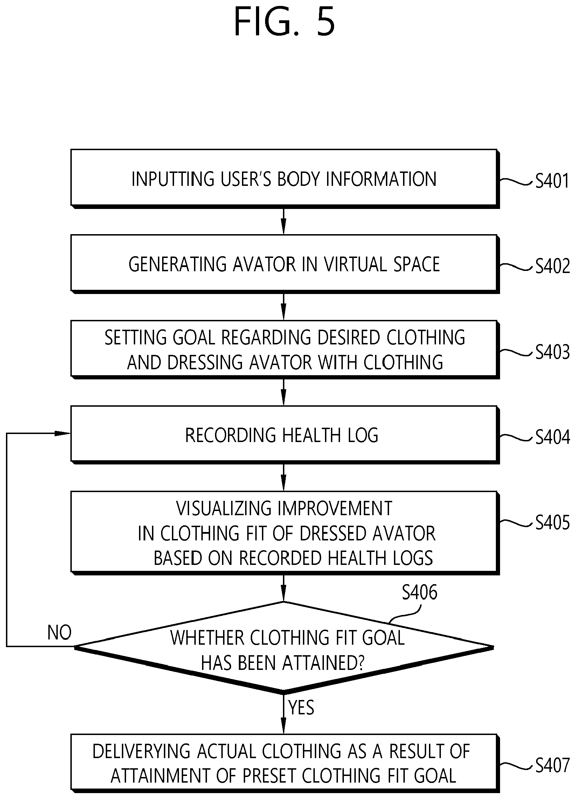

FIG.5 is a flowchart illustrating an operation of attaining a clothing fit goal using an avatar in a virtual space by a mobile healthcare service apparatus according to an embodiment of the present disclosure.

In operation S401, a mobile healthcare service apparatus 100 receives a user's body information.

In operation S402, the mobile healthcare service apparatus 100 creates an avatar tailored to the received user's body information in a virtual space. Here, at least one avatar may be created in a virtual space tailored to the user's body conditions. Alternatively, at least one avatar may be created based on desired clothing.

In operation S403, the mobile healthcare service apparatus 100 may set a goal regarding the clothing the user wishes to wear, generate virtual clothing based on the set clothing objective, and display the avatar adorned with the created virtual clothing to the user.

In operation S404, the mobile healthcare service apparatus 100 may collect and record health logs related to the user's health, such as the user's diet and exercise. Here, the user may set desired clothing as a goal and keep a health log (e.g., dietary record, exercise record, etc.).

In operation S405, the mobile healthcare service apparatus 100 may visualize improvements in the clothing fit of an avatar dressed with virtual clothing based on recorded health logs. For example, the mobile healthcare service apparatus 100 may improve the clothing fit of the avatar dressed with the virtual clothing as the recorded health logs approach a preset clothing fit goal. Alternatively, the mobile healthcare service apparatus 100 may visually demonstrate gradual improvements in the fit of virtual clothing on the avatar in the virtual space as the user's health logs accumulate.

In operation S406, the mobile healthcare service apparatus 100 determines whether a preset clothing fit goal has been attained based on the recorded health logs.

In operation S407, when the preset clothing fit goal is attained based on the recorded health logs, the mobile healthcare service apparatus 100 may deliver actual clothes as a result of the attainment of the preset clothing fit goal. This is to motivate a user, who has attained the user's targeted clothing fit or succeed in recording health log missions, by delivering the intended clothes the user aimed for. If it fails to attain the preset clothing fit goal based on the recorded health logs, the mobile healthcare service apparatus 100 performs operation S404 of recording health logs.

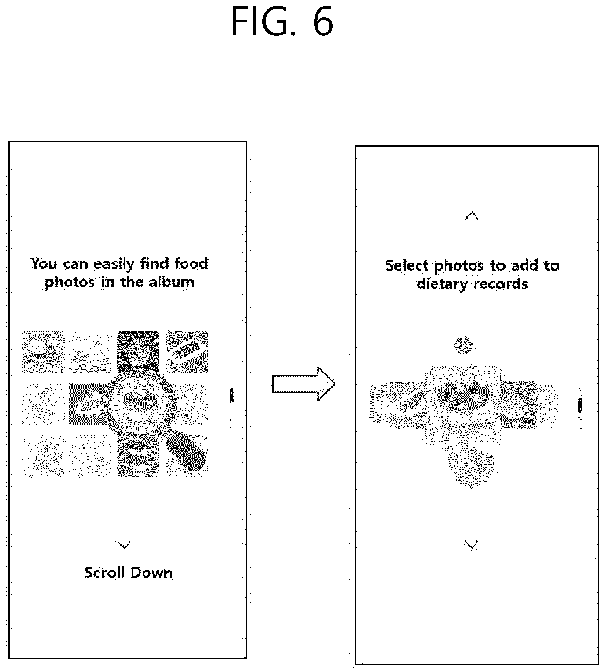

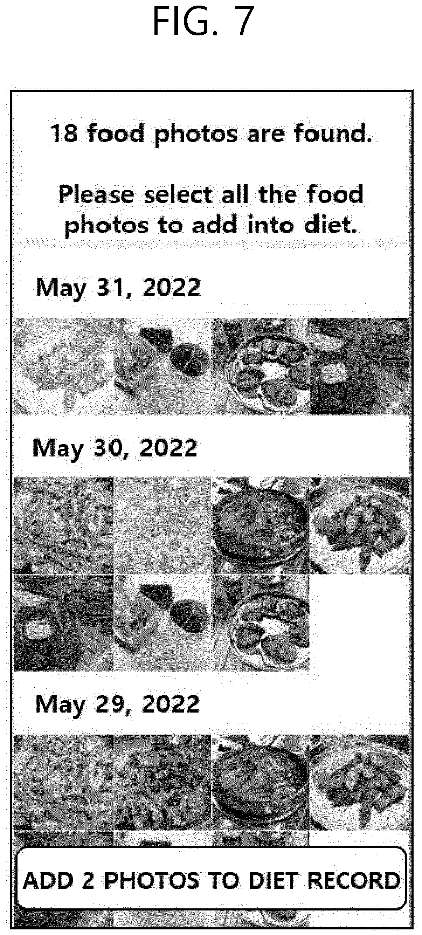

FIGS. 6 and 7 are diagrams showing examples of diet management and analysis by a mobile healthcare service apparatus according to an embodiment of the present disclosure.

As shown in FIG. 6, the mobile healthcare service apparatus 100 according to an embodiment of the present disclosure may automatically find food photos among photos stored in an album. The mobile healthcare service apparatus 100 may receive a user's selection of a photo to add to a dietary record from among the found photos. The mobile healthcare service apparatus 100 may analyze calories and nutrients (e.g., carbohydrates, proteins, fats, etc.) by analyzing the selected photo of food to add to a dietary record.

As shown in FIG. 7, the mobile healthcare service apparatus 100 according to an embodiment of the present disclosure may perform a food photo search function. The mobile healthcare service apparatus 100 may display food photos found in the album to the user and add food photos (for example, two food photos) to record to a diet.

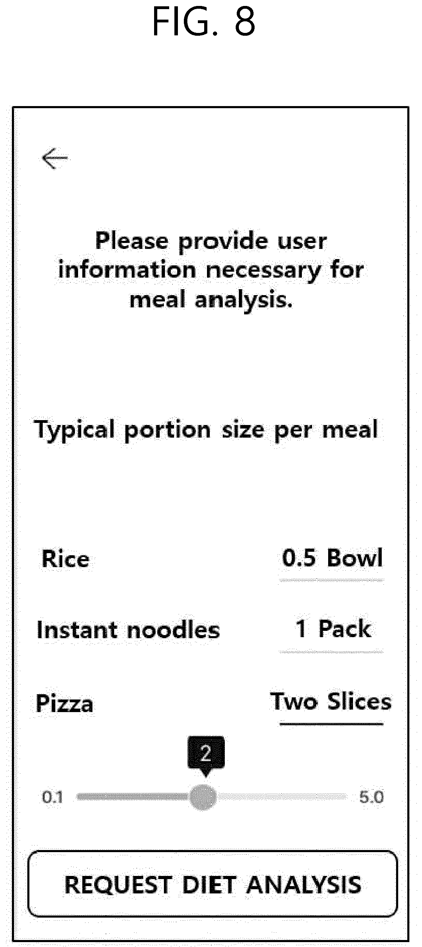

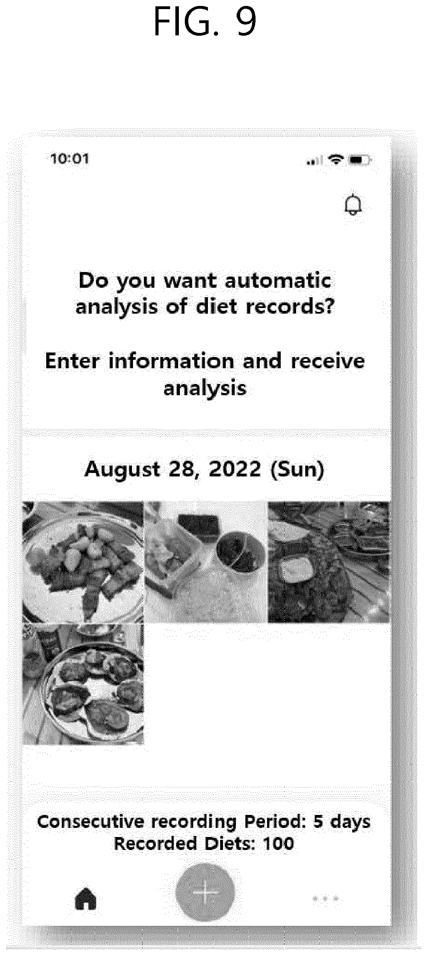

FIGS. 8 and 9 are diagrams showing an example of requesting dietary analysis by a mobile healthcare service apparatus according to an embodiment of the present disclosure.

As shown in FIG. 8, the mobile healthcare service apparatus 100 according to an embodiment of the present disclosure may receive user information (such as date of birth, gender, typical portion size per meal, etc.) necessary for dietary analysis. Here, the mobile healthcare service apparatus 100 may receive intake quantities in food units corresponding to various food types (such as a bowl of rice, a packet of ramen, a slice of pizza, etc.) for the typical portion size per meal. In addition, the mobile healthcare service apparatus 100 may analyze the diet upon the user's dietary analysis request.

As shown in FIG. 9, the mobile healthcare service apparatus 100 according to an embodiment of the present disclosure may automatically analyze an input dietary record from calories to nutrients and provide a result of the analysis to the user. At this point, the mobile healthcare service apparatus 100 may mark a food photo or dietary record being analyzed as "under analysis".

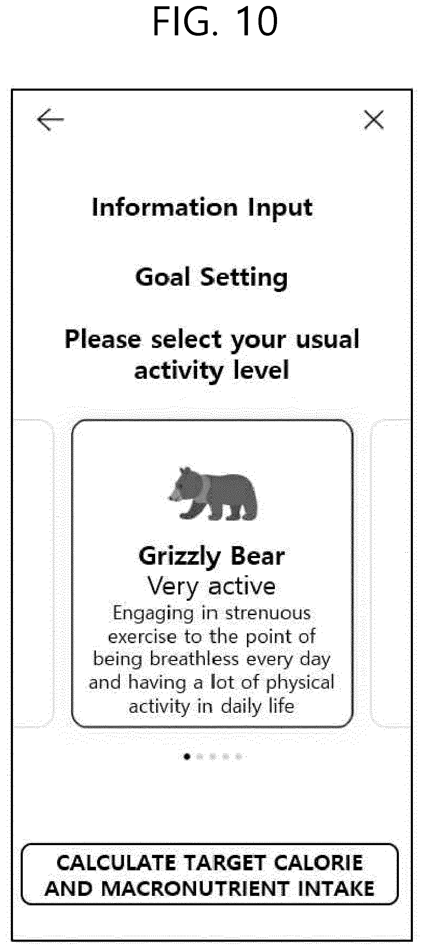

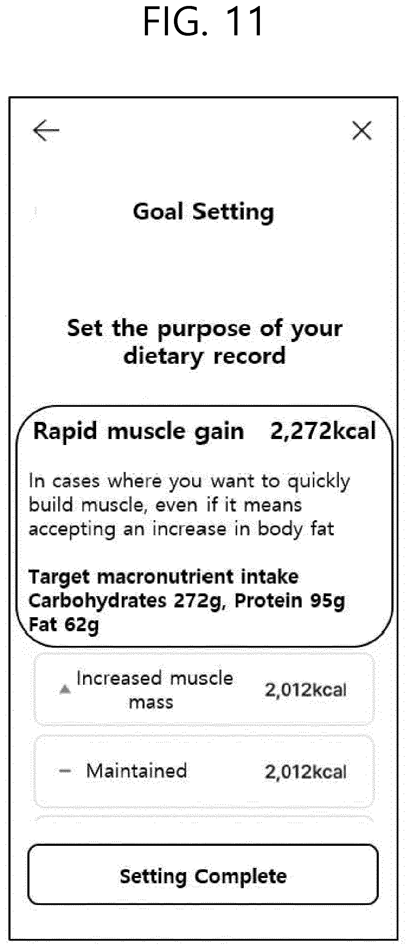

FIGS. 10 and 11 are diagrams showing an example of setting a target calorie and nutrient by a mobile healthcare service apparatus according to an embodiment of the present disclosure.

As shown in FIG. 10, the mobile healthcare service apparatus 100 according to an embodiment of the present disclosure may receive user information (e.g., height, weight, daily physical activity, etc.) required for calculating a target intake. Here, the daily physical activity may be classified into categories such as highly active, moderately active, or sedentary. The mobile healthcare service apparatus 100 may calculate a target calorie intake and target nutrients based on an input of user information.

As shown in FIG. 11, the mobile healthcare service apparatus 100 according to an embodiment of the present disclosure may receive a purpose of diet recording from a user. For example, the purpose of diet recording may include purposes such as rapid muscle gain, muscle gain, or muscle maintenance. For example, if the diet recording purpose is set to rapid muscle gain in user information, the mobile healthcare service device 100 may suggest intaking the required daily calories for rapid muscle gain, such as 2,272 kcal, and also display the nutrient ratios needed for the intake, including carbohydrates, proteins, fats, etc. In addition, the mobile healthcare service apparatus 100 may display an additional available intake, whether the calorie goal has been attained, and a habit-building feature creation function through participation in a diet recording challenge.

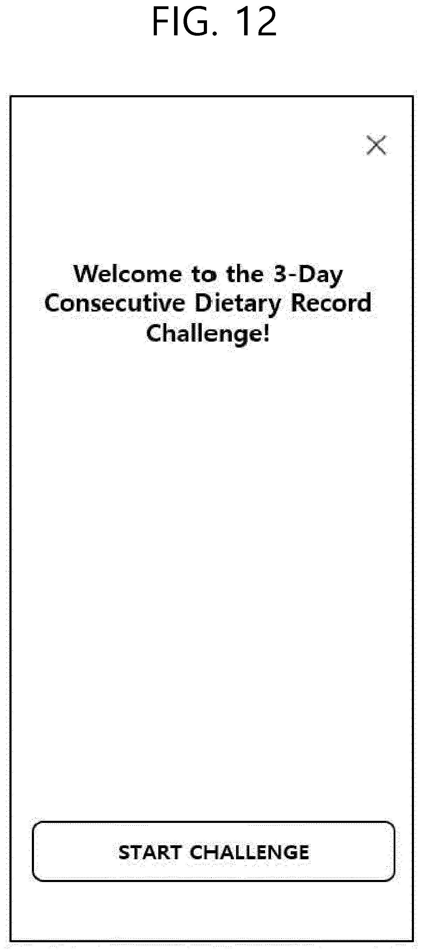

FIG. 12 is a diagram showing an example of a diet recording challenge operation by a mobile healthcare service apparatus according to an embodiment of the present disclosure.

As shown in FIG. 12, the mobile healthcare service apparatus 100 according to an embodiment of the present disclosure may inform a user participating in the diet recording challenge about the importance of dietary records and set diet recording alerts to aid in the successful completion of the challenge. For example, the mobile healthcare service apparatus 100 may allow a user to set a dietary record notification needed for the diet recording challenge, such as morning 9 AM, noon 1 PM, evening 7 PM, and snack at 3 PM.

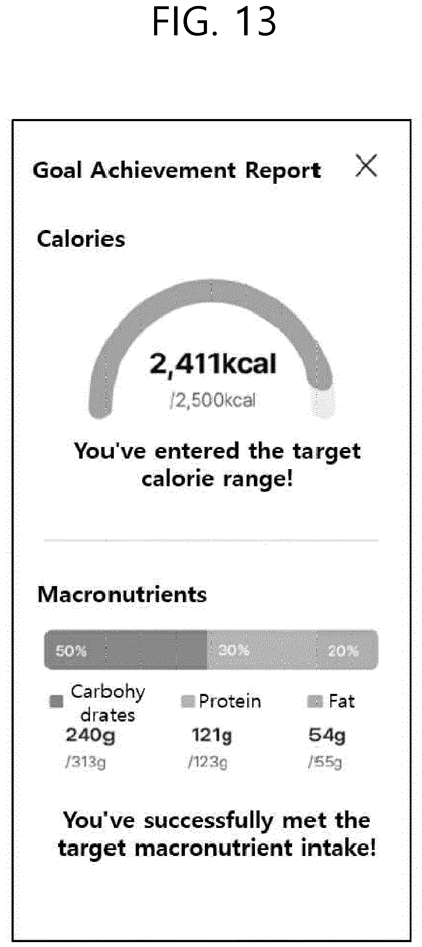

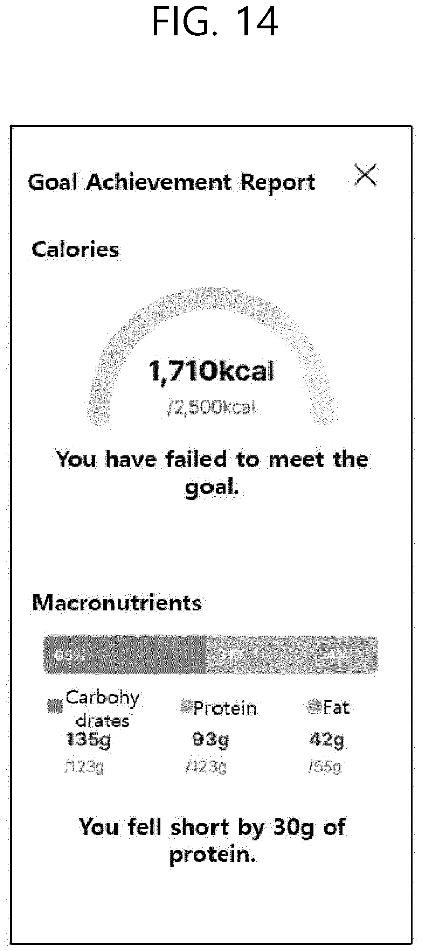

FIGS. 13 and 14 are diagrams showing an example of a goal attainment report provided by a mobile healthcare service apparatus according to an embodiment of the present disclosure.

As shown in FIG. 13, the mobile healthcare service apparatus 100 according to an embodiment of the present disclosure may display a target calorie range included in a diet recording challenge and indicate, for each day of performing the challenge, whether the goal is attained, missed, or pending. The mobile healthcare service apparatus 100 may provide a goal attainment report indicating whether the target calorie range has been reached and the likelihood of success in the diet recording challenge (e.g., rapid weight loss achievable soon). The goal attainment report may include intake amounts based on nutrient ratios and the attainment status of nutrient target amounts.

As shown in FIG. 14, the mobile healthcare service apparatus 100 according to an embodiment of the present disclosure may display, during a specific period, the dates in which goals were not attained. The mobile healthcare service apparatus 100 may display a reason for failing to attain a goal aligned with a diet recording challenge (such as "consumed 30g less protein") and also present relevant health management information. In addition, the mobile healthcare service apparatus 100 may provide a photo of food if the goal of the diet recording challenge is not attained.

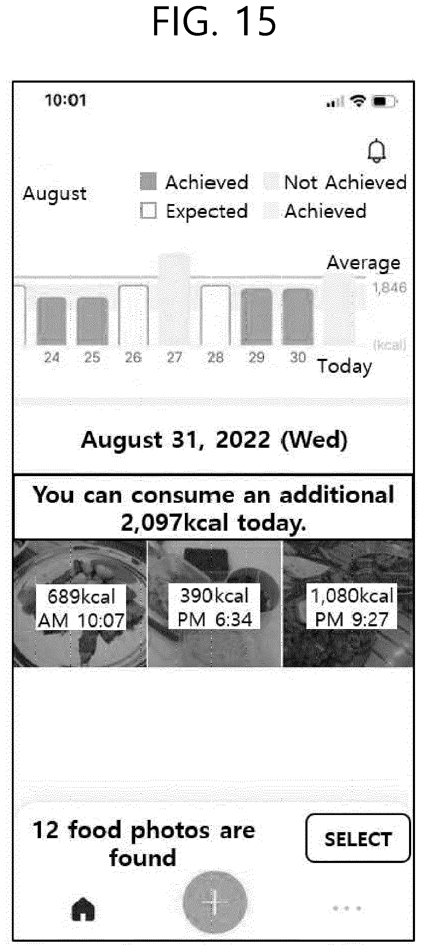

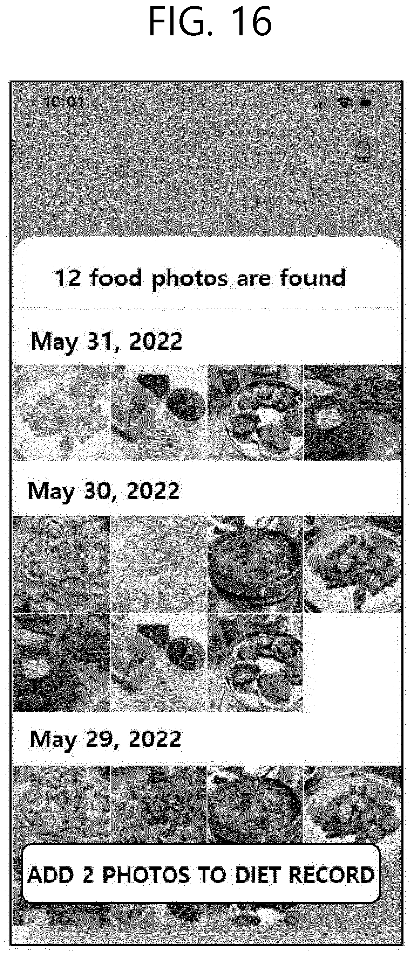

FIGS. 15 and 16 are diagrams showing an example of a food photo search operation in an album by a mobile healthcare service apparatus according to an embodiment of the present disclosure.

As shown in FIG. 15, the mobile healthcare service apparatus 100 according to an embodiment of the present disclosure may find food photos by detecting only for photos of food taken in the album. For example, the mobile healthcare service apparatus 100 may find 12 food photos among 123 photos.

As shown in FIG. 16, the mobile healthcare service apparatus 100 according to an embodiment of the present disclosure may receive a user's selection of a food photo to add to a dietary record from among found food photos. For example, the mobile healthcare service apparatus 100 may add 2 food photos out of 12 food photos to a dietary record. When a food photo is added to the diet, the mobile healthcare service apparatus 100 may add the food photo for that date and analyze or update the dietary record for that date based on the added food photo.

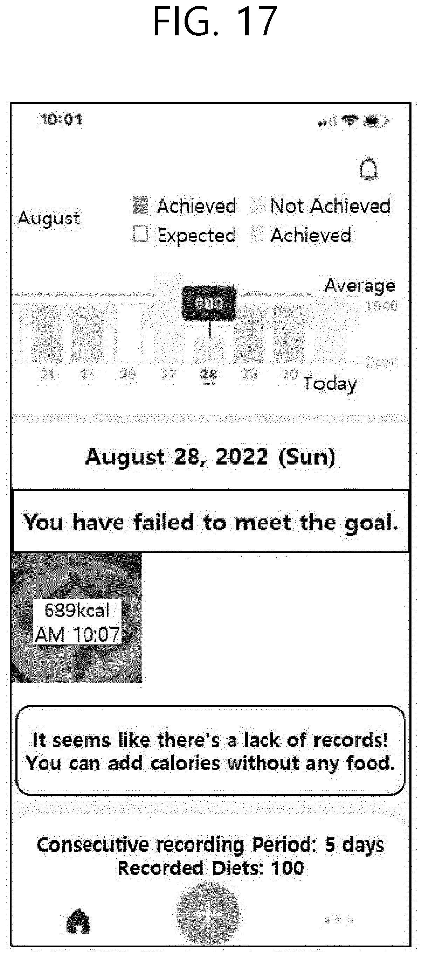

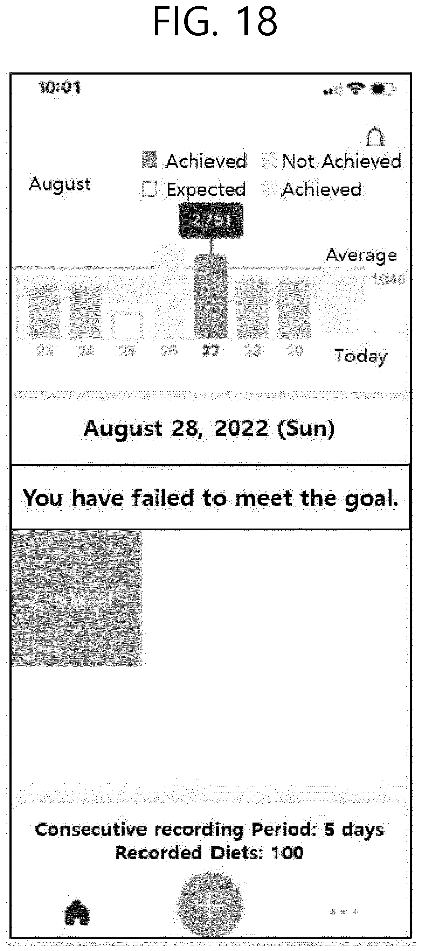

FIGS. 17 and 18 are diagrams showing an example of displaying a calorie flow and expected calorie intake by a mobile healthcare service apparatus according to an embodiment of the present disclosure.

As shown in FIG. 17, when a user fails to attain a diet recording challenge goal on a date where any food photo is not captured or added, the mobile healthcare service apparatus 100 according to an embodiment of the present disclosure may maintain a graph flow by adding calories without food. When any food photo is not added, the mobile healthcare service apparatus 100 may display, to a user, a message indicating that calories can be added without a food photo. In doing so, the mobile healthcare service apparatus 100 may receive additions of various types of calories (such as 'I ate like a bird", "I ate a bit less", "I ate as usual", "I ate quite a lot", "it was a cheat day", etc.).

As shown in FIG. 18, when there is no dietary record, the mobile healthcare service apparatus 100 according to an embodiment of the present disclosure may record calories entered by a user as a day-to-day calorie graph and associate the calories with calorie intake based on food consumption. Alternatively, even on dates where there is no quantity or calorie input through food photos, the mobile healthcare service apparatus 100 may maintain a calorie graph flow by predicting calorie intake based on previous patterns of calorie intake, etc. Even on dates where there is no record, the mobile healthcare service apparatus 100 may predict an expected quantity and display the expected quantity to the user, and later adjust the calorie intake based on the user's input.

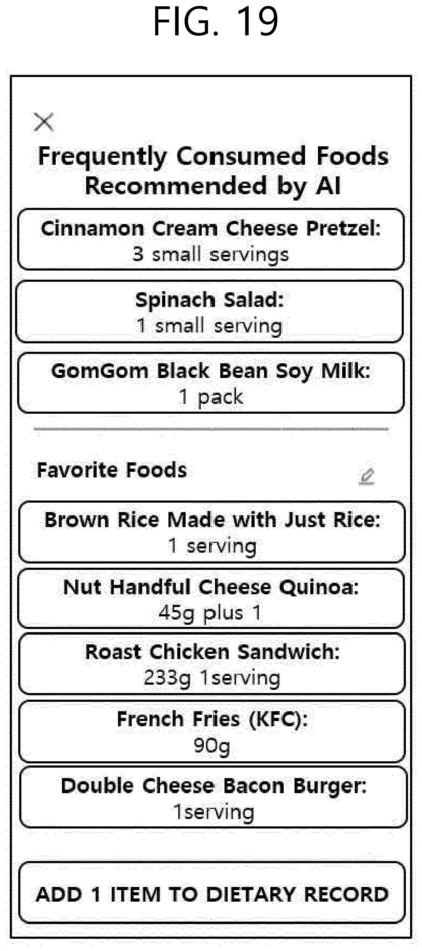

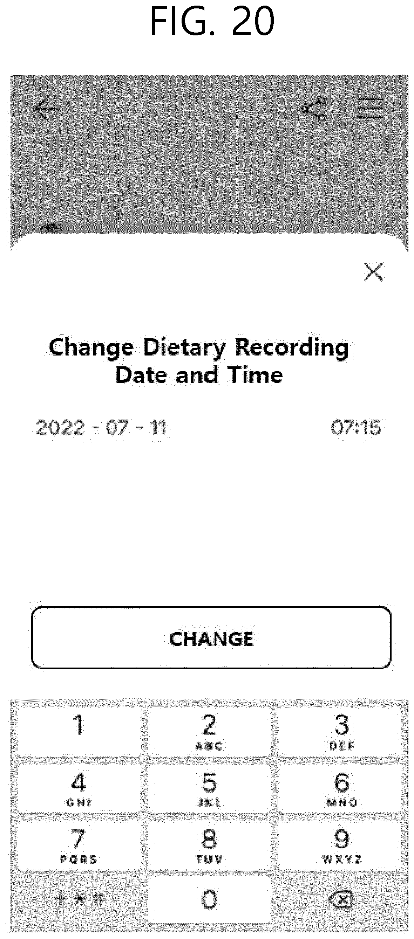

FIGS. 19 and 20 are diagrams illustrating an example of quickly recording a diet with favorite foods by a mobile healthcare service apparatus according to an embodiment of the present disclosure.

As shown in FIG. 19, the mobile healthcare service apparatus 100 according to an embodiment of the present disclosure can quickly record a diet with favorite foods. For example, the mobile healthcare service apparatus 100 can recommend frequently eaten foods or favorite foods through AI artificial intelligence model recommendation. When a user selects a frequently eaten food recommended by AI, the mobile healthcare service apparatus 100 may also receive the user's selection of a unit of the selected food and add the selected unit to a dietary record.

As shown in FIG.20, the mobile healthcare service apparatus 100 according to an embodiment of the present disclosure may change details of a recorded diet and the diet recording date or time upon the user's request for modification.

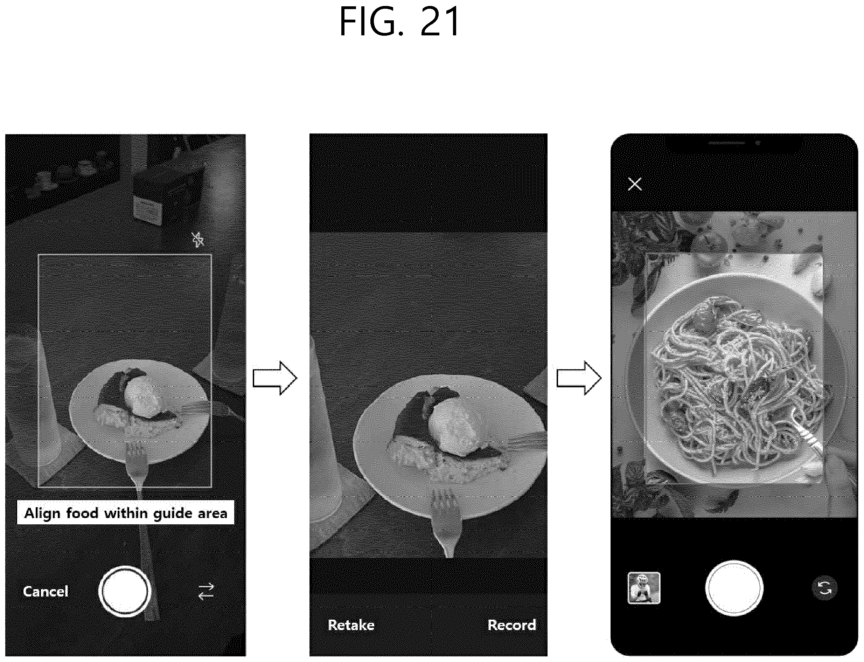

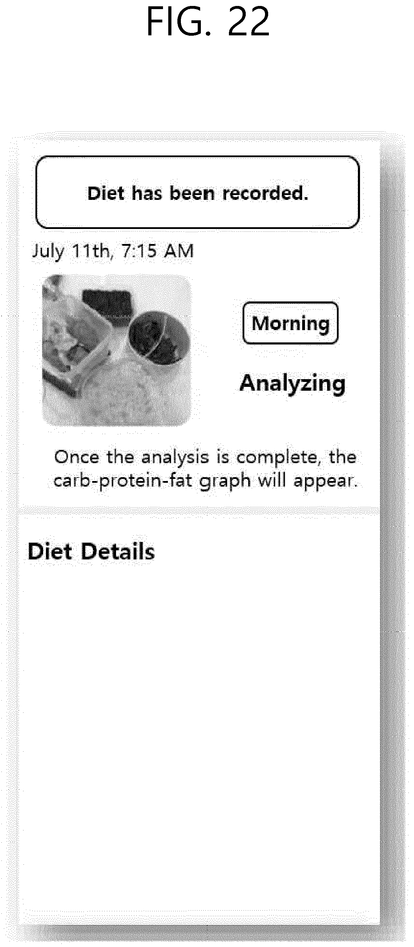

FIGS. 21 and 22 are diagrams showing an example of a simple diet recording operation using photos by a mobile healthcare service apparatus according to an embodiment of the present disclosure.

As shown in FIG. 21, the mobile healthcare service apparatus 100 according to an embodiment of the present disclosure may easily record a diet using a food photo by taking a photo where food is located. Here, the mobile healthcare service apparatus 100 may display a guide area so that the user can algin food accurately within the guide area so as to position food within a food photo. Alternatively, the mobile healthcare service apparatus 100 may display a food area when any food is detected on a photoshoot screen.

As shown in FIG. 22, when a food photo is selected from an album, the mobile healthcare service apparatus 100 according to an embodiment of the present disclosure may add the selected food photo as a dietary record, analyze the dietary record, and provide information on diet details to a user.

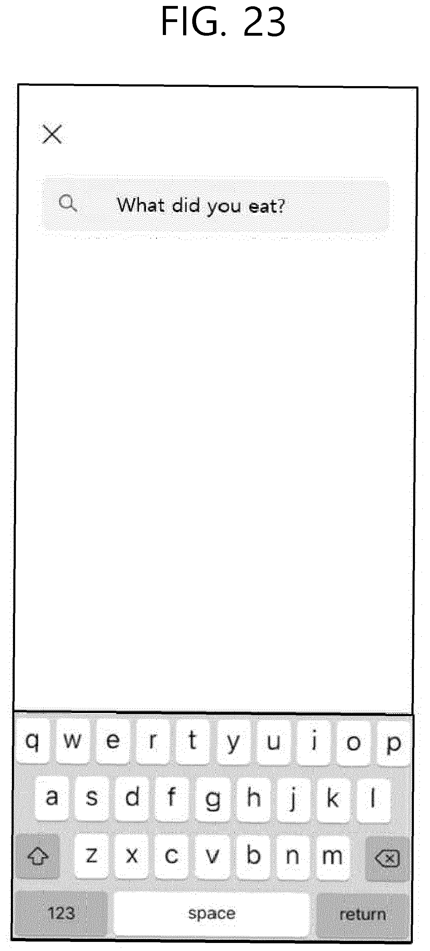

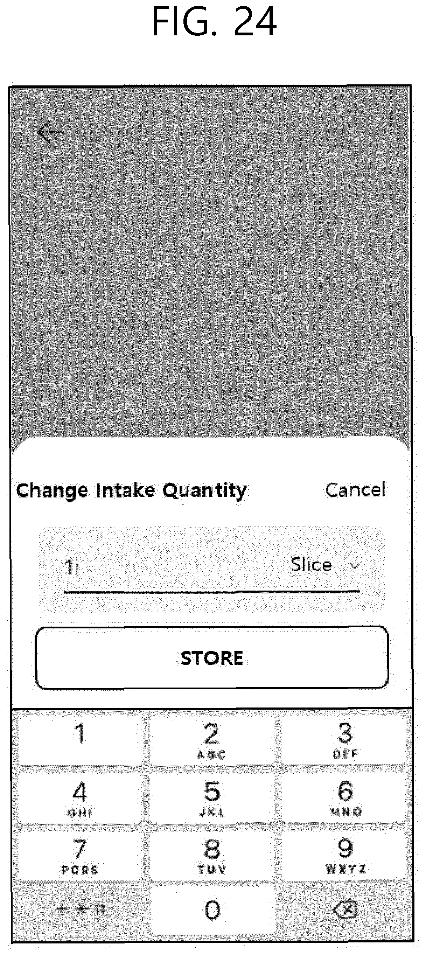

FIGS. 23 and 24 are diagrams showing an example of searching for and recording a diet by a mobile healthcare service apparatus according to an embodiment of the present disclosure.

As shown in FIG. 23, the mobile healthcare service apparatus 100 according to an embodiment of the present disclosure may provide a food search screen to a user and directly record a found food into a diet.

As shown in FIG. 24, the mobile healthcare service apparatus 100 according to an embodiment of the present disclosure may provide a function for changing the date and quantity of a diet including the found food.

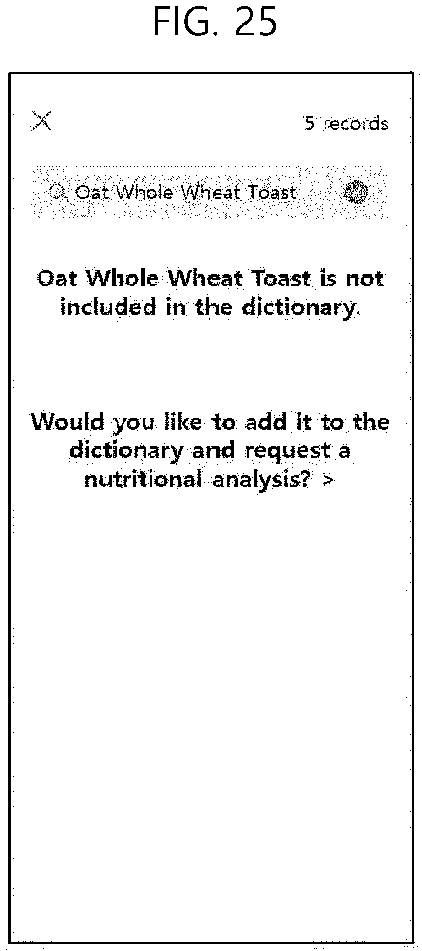

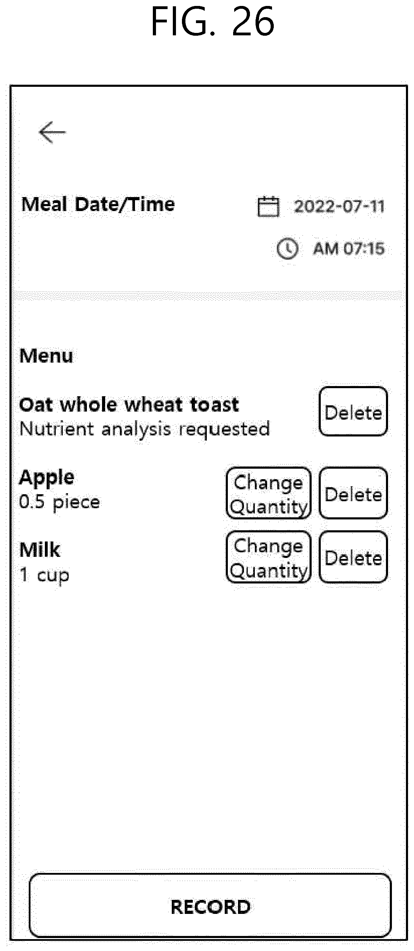

FIGS. 25 and 26 are diagrams showing an example of an operation of requesting nutrient analysis of an unfound item by a mobile healthcare service apparatus according to an embodiment of the present disclosure.

As shown in FIG. 25, the mobile healthcare service apparatus 100 according to an embodiment of the present disclosure may receive a request to analyze nutrients for an item not found. When there is a food (e.g., oat whole wheat toast, etc.) that is not found in an item list, the mobile healthcare service apparatus 100 may provide a message indicating that the food is not included in the food dictionary, and receive a request for nutrient analysis.

As shown in FIG. 26, upon receiving a request for nutrient analysis for an item not found, the mobile healthcare service apparatus 100 may perform nutrient analysis and provide a result of the nutrient analysis to a user.

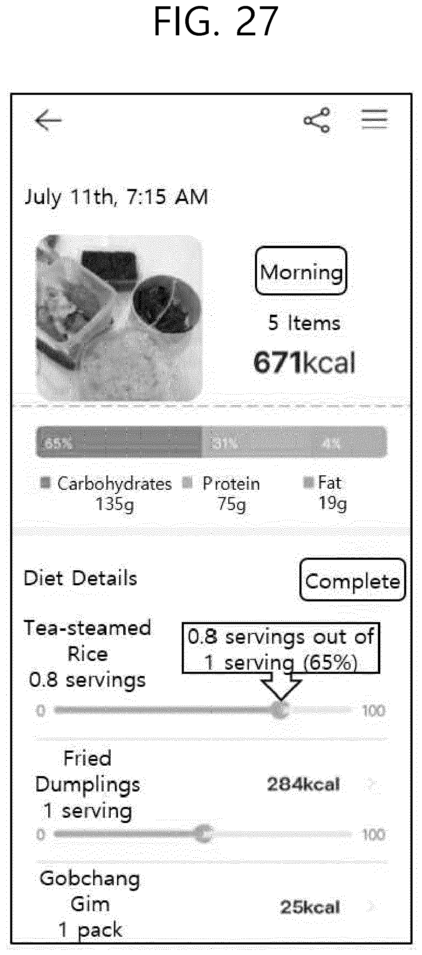

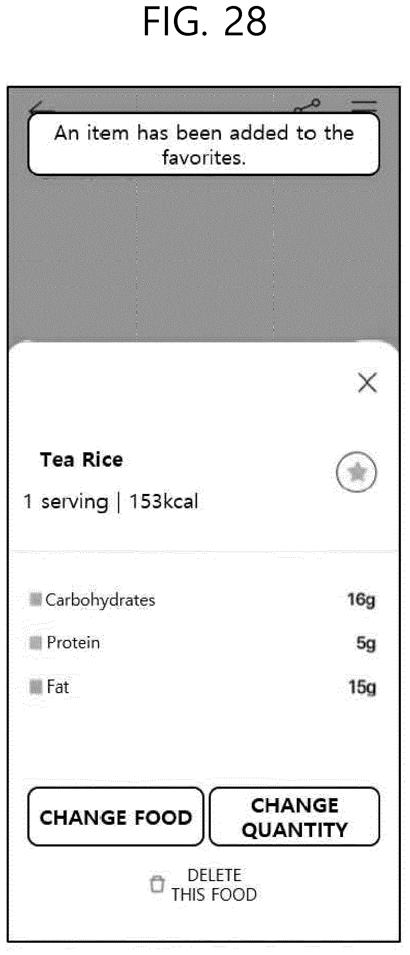

FIGS. 27 and 28 are diagrams showing examples of a result of food photo analysis by a mobile healthcare service apparatus according to an embodiment of the present disclosure.

As shown in FIG. 27, the mobile healthcare service apparatus 100 according to an embodiment of the present disclosure may provide a user with a slider to allow the user to adjust a quantity for each of dietary details. The mobile healthcare service apparatus 100 may easily change a quantity for a food photo using the slider.

As shown in FIG. 28, the mobile healthcare service apparatus 100 according to an embodiment of the present disclosure may provide a button for adding a favorite food. The mobile healthcare service apparatus 100 may add a favorite food into a diet by clicking the button.

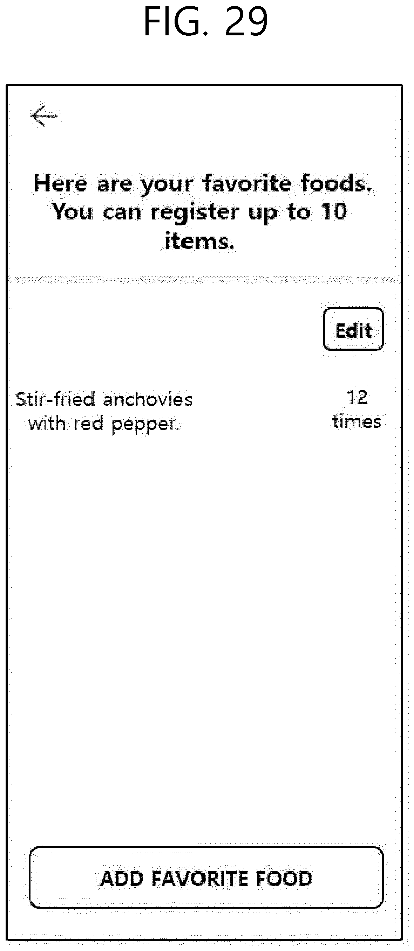

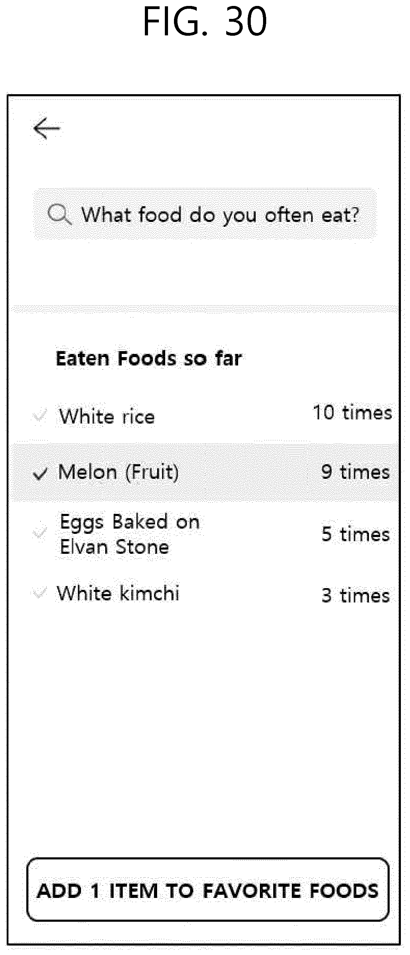

FIGS. 29 and 30 are diagrams showing an example of an operation of editing favorite foods by a mobile healthcare service apparatus according to an embodiment of the present disclosure.

As shown in FIG. 29, the mobile healthcare service apparatus 100 according to an embodiment of the present disclosure may display a user's favorite food, if any. In addition, the mobile healthcare service apparatus 100 may display frequently consumed foods along with the number of times the foods have been eaten.

As shown in FIG. 30, the mobile healthcare service apparatus 100 according to an embodiment of the present disclosure displays the number of foods eaten so far to the user. When the user selects a food item to add to the favorite food, the mobile healthcare service apparatus 100 may add the selected food as a favorite food.

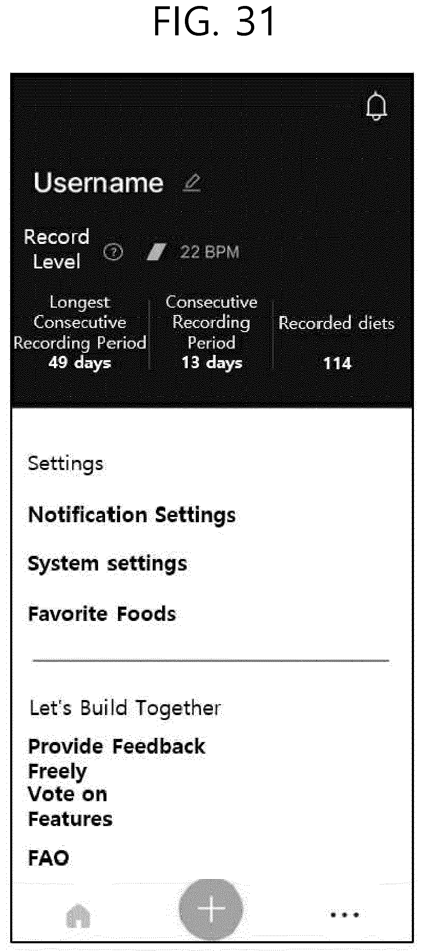

FIG.31 is a diagram showing an example of menu analysis settings by a mobile healthcare service apparatus according to an embodiment of the present disclosure.

As shown in FIG. 31, the mobile healthcare service apparatus 100 according to an embodiment of the present disclosure may display notification settings, system settings, favorite foods, etc. The mobile healthcare service apparatus 100 may display notifications for calorie flow interruption, food photo search, food reanalysis completion, and automatic diet analysis completion.

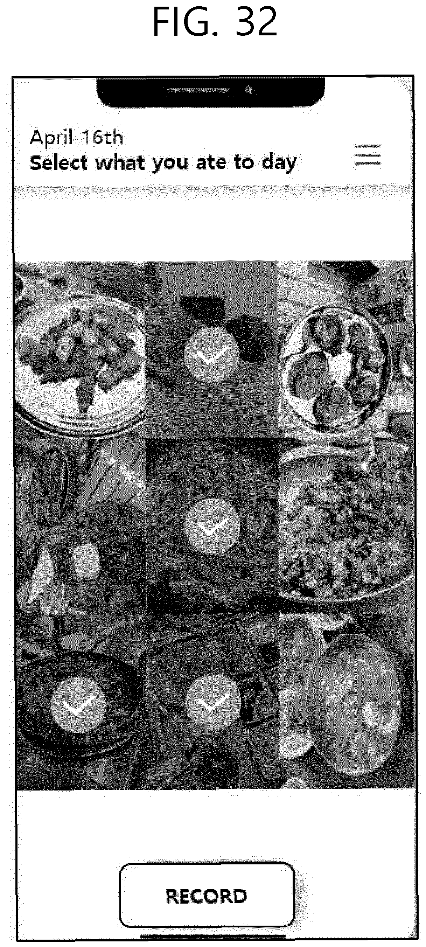

FIG.32 is a diagram showing an example of a camera album by a mobile healthcare service apparatus according to an embodiment of the present disclosure.

As shown in FIG. 32, the mobile healthcare service apparatus 100 according to an embodiment of the present disclosure may provide an operation of selecting consumed food, an operation of performing dietary analysis, and an example of a food analysis result. The mobile healthcare service apparatus 100 may display photos of food to add to the album during the operation of selecting consumed food. The mobile healthcare service apparatus 100 may display a type and calories for each food analyzed during the operation of performing dietary analysis The mobile healthcare service apparatus 100 may display calories and nutrients for each type of food eaten in a food analysis result.

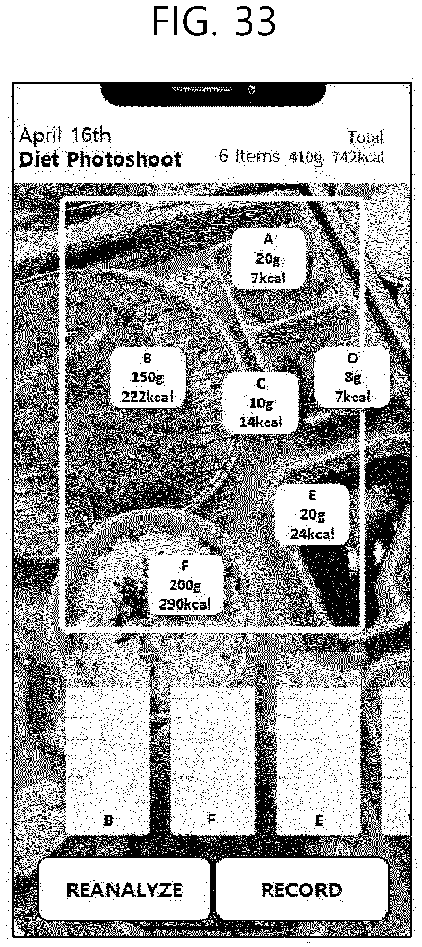

FIG.33 is a diagram showing an example of a camera AR operation by a mobile healthcare service apparatus according to an embodiment of the present disclosure.

As shown in FIG. 33, the mobile healthcare service apparatus 100 according to an embodiment of the present disclosure may display a type and calories for each food in AR during a meal photographing operation. In addition, the mobile healthcare service apparatus 100 may display an intake bar at the bottom to adjust the intake of food, and when the user adjusts the intake bar, the adjusted intake may be recorded as the intake of the corresponding food.

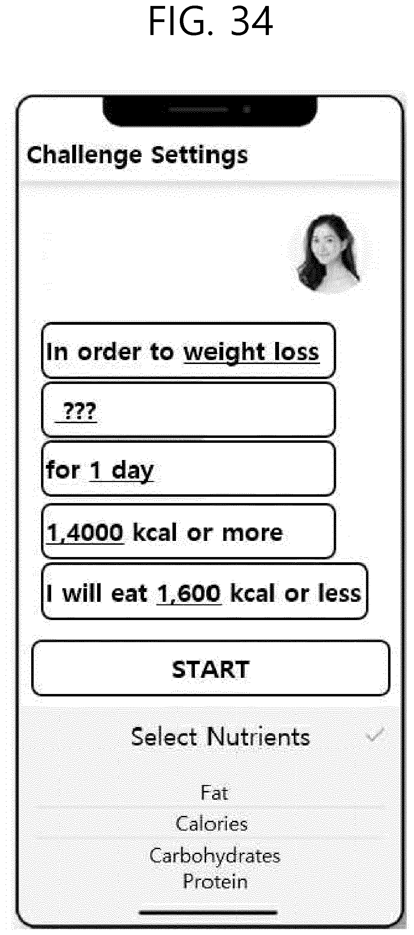

FIG.34 is a diagram showing an example of individual and team competition actions for a diet challenge by a mobile healthcare service apparatus according to an embodiment of the present disclosure.

As shown in FIG. 34, the mobile healthcare service apparatus 100 according to an embodiment of the present disclosure may receive input regarding nutrients to be tracked and a goal. The mobile healthcare service apparatus 100 may communicate with family members about the diet challenge through a chat window. The mobile healthcare service apparatus 100 may provide a ranking of goal attainments by family member. For each family member, a goal, an achievement rate, and cumulative attainments may be displayed. The mobile healthcare service apparatus 100 may display the success rate for each team participating in the dietary challenge or rankings among participating teams based on success rates or cumulative attainments.

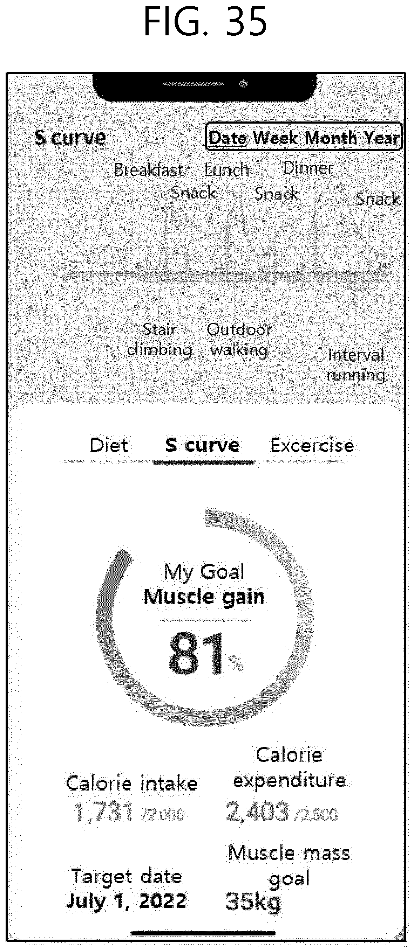

FIG.35 is a diagram showing an example of an analysis S curve by a mobile healthcare service apparatus according to an embodiment of the present disclosure.

As shown in FIG. 35, the mobile healthcare service apparatus 100 according to an embodiment of the present disclosure may display a graph with intake calories, expended calories, and net calories (calories consumed minus calories burned) to help a user attain his or her goal (e.g., weight loss). The mobile healthcare service apparatus 100 may display a dietary record showing a photo of food recorded in the diet. The mobile healthcare service apparatus 100 may display a dietary record showing a photo of food recorded in the diet. The mobile healthcare service apparatus 100 may display the types of exercises the user engages in (such as walking, stair climbing, running, fitness, home workouts, etc.) along with the respective activity levels for the exercises.

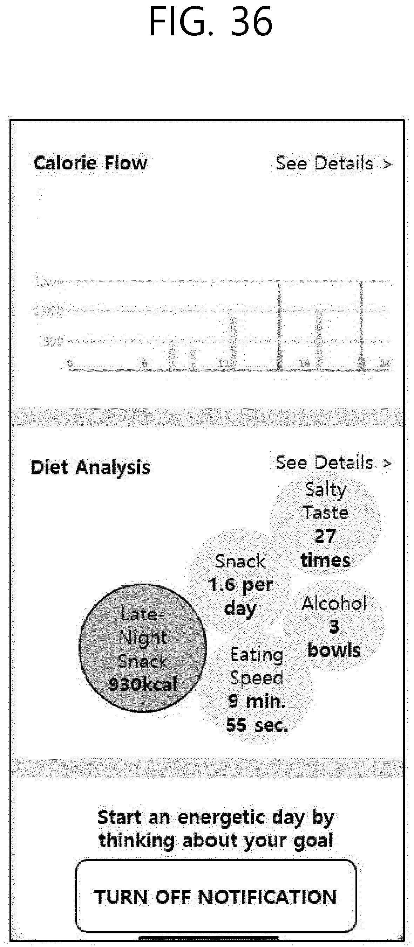

FIG.36 is a diagram showing an example of an eating habits analyzing operation by a mobile healthcare service apparatus according to an embodiment of the present disclosure.

As shown in FIG. 36, the mobile healthcare service apparatus 100 according to an embodiment of the present disclosure may display a dietary analysis result that include overall calorie intake from yesterday, calories consumed per hour, and the flow of food or calories throughout the day. The mobile healthcare service apparatus 100 may provide a daily average calorie intake trend calculated based on cumulative dietary records, a dietary analysis result, etc.

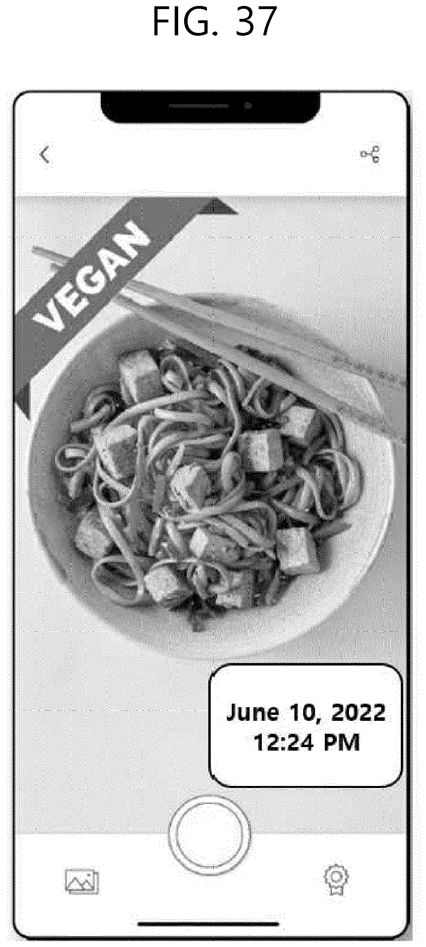

FIG.37 is a diagram showing an example of a vegetarianism authentication operation by a mobile healthcare service apparatus according to an embodiment of the present disclosure.

As shown in FIG. 37, the mobile healthcare service apparatus 100 according to an embodiment of the present disclosure may receive an input regarding a vegetarian lifestyle goal, motivation for vegetarianism, etc. and perform a vegetarianism authentication operation. The mobile healthcare service apparatus 100 may provide vegetarianism authentication for a photo of vegetable food. The mobile healthcare service apparatus 100 may display a vegetarianism status by recorded vegetarianism period and the badges attained based on vegetarianism authentication results. In addition, the mobile healthcare service apparatus 100 may also provide an authentication gallery for vegetarianism authentication.

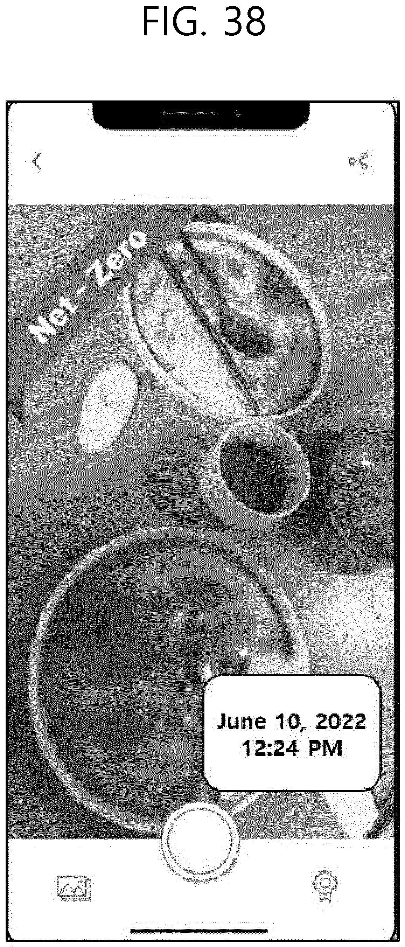

FIG.38 is a diagram showing an example of a net-zero authentication operation by a mobile healthcare service apparatus according to an embodiment of the present disclosure.

As shown in FIG. 38, the mobile healthcare service apparatus 100 according to an embodiment of the present disclosure may perform net-zero authentication by receiving a net-zero lifestyle goal and a greenhouse gas reduction strategy for achieving net-zero emissions. The mobile healthcare service apparatus 100 may perform net-zero authentication for a food photo where there is no food leftovers. The mobile healthcare service apparatus 100 may display a net-zero status by recorded net-zero period and badges attained based on net-zero authentication results. In addition, the mobile healthcare service apparatus 100 may also provide an authentication gallery for net-zero authentication.

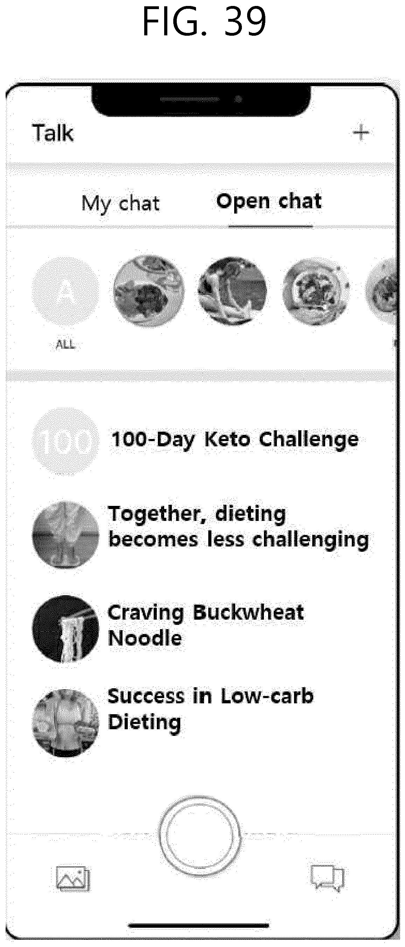

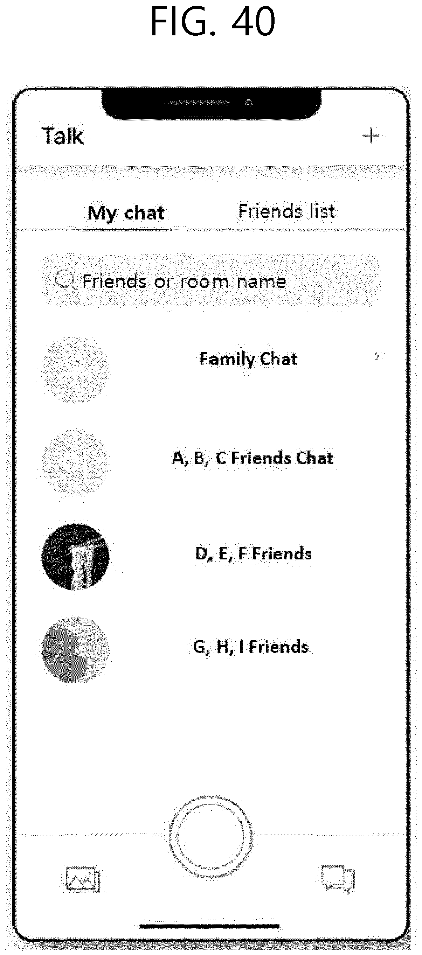

FIGS. 39 and 40 are diagrams showing examples of open chat and friend chat operations by a mobile healthcare service apparatus according to an embodiment of the present disclosure.

As shown in FIG. 39, the mobile healthcare service apparatus 100 according to an embodiment of the present disclosure may provide an open chat so that a user can communicate with other users participating in a diet challenge. The mobile healthcare service apparatus 100 may allow chatting for each open chat and analyze and provide calories and nutrients for a food photo posted by the user. In addition, the mobile healthcare service apparatus 100 may also provide a food gallery for each open chat.

As shown in FIG. 40, the mobile healthcare service apparatus 100 according to an embodiment of the present disclosure may provide a friend chat so that a user can communicate with friends or family members participating in a diet challenge. The mobile healthcare service apparatus 100 may allow the user to chat with each friend, and analyze and provide calories and nutrients for a diet photo posted by the user. In addition, the mobile healthcare service apparatus 100 may also provide a food gallery for each friend chat.

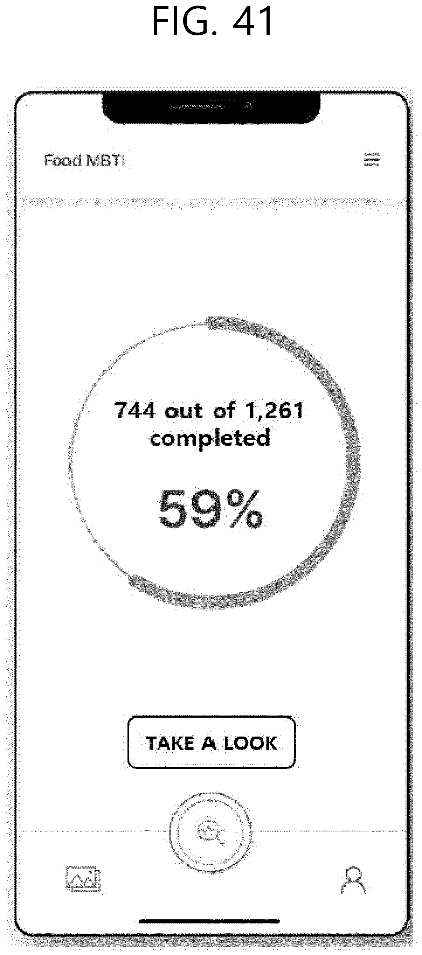

FIG.41 is a diagram showing an example of food MBTI by a mobile healthcare service apparatus according to an embodiment of the present disclosure.

As shown in FIG. 41, the mobile healthcare service apparatus 100 according to an embodiment of the present disclosure may provide food MBTI to the user. The mobile healthcare service apparatus 100 may analyzes all food photos in the album and, based on the analysis results, provide meal type results (such as a dedicated carb-lover, etc.), big data categorized by meal type, and a user's eating preferences (such as the ranking of frequently consumed foods). The mobile healthcare service apparatus 100 may compare the results of meal analysis with friends to provide food MBTI compatibility results, recommended foods based on the compatibility, and information about recommended restaurants.

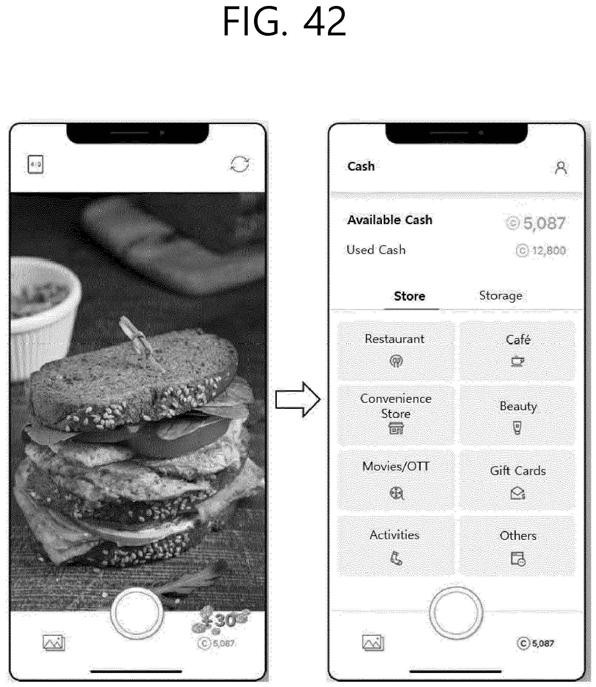

FIG.42 is a diagram showing an example of food cash by a mobile healthcare service apparatus according to an embodiment of the present disclosure.

As shown in FIG.42, when a user records a food photo into a diet, the mobile healthcare service apparatus 100 may provide food cash rewards for each recorded food photo or for a specific food included in the food photo. The mobile healthcare service apparatus 100 may provide food cash based on an attainment status of a diet challenge goal (such as vegetarianism, zero food waste, etc.) for each daily dietary record. In addition, the mobile healthcare service apparatus 100 may provide a food cash status showing available cash and used cash, or a store where food cash can be used.

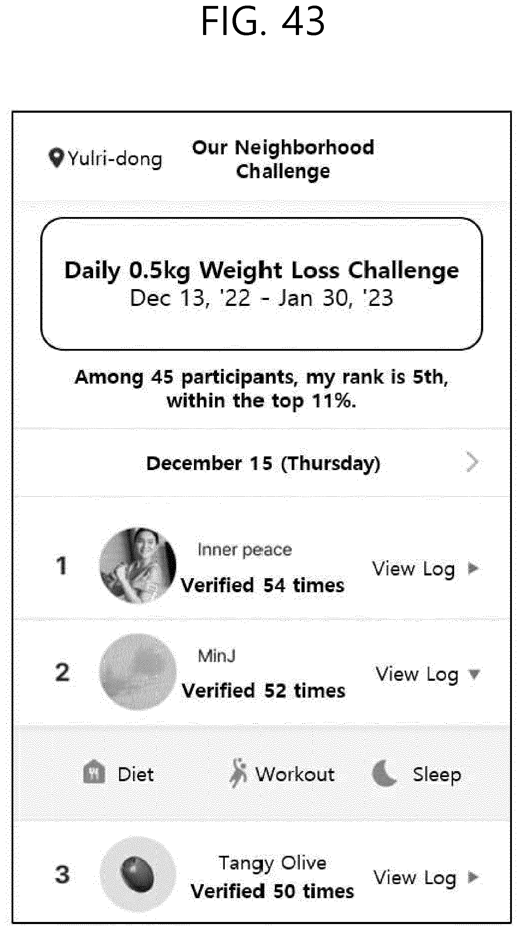

FIG.43 is a diagram showing an example of a personal challenge and group challenge operation by a mobile healthcare service apparatus according to an embodiment of the present disclosure.

As shown in FIG. 43, for goals of challenges that a user is participating, the mobile healthcare service apparatus 100 according to an embodiment of the present disclosure may provide the user with the certification status of other users participating in a challenge, the number of challenge certifications, and log data of other users' challenge progress.

The mobile healthcare service apparatus 100 may provide group-specific competitive status regarding the goal of a challenge participated in by the groups (for example, a challenge to do 100 squats daily) (for example, messages indicating if the user's group is leading or if more efforts are needed compared to other groups). The mobile healthcare service apparatus 100 may provide a comment window for each group participating in the group challenge goal to enable communication by group or between groups.

In this regard, according to embodiments, the mobile healthcare service apparatus 100 may generate a goal attainment algorithm using health logs of individuals who have attained their goals, and may provide step-by-step guidelines and missions for goal attainment to individuals with similar conditions and goals. At this point, rather than simple step-by-step guidelines and missions, the mobile healthcare service apparatus 100 may produce and provide to users the most effective step-by-step guidelines and missions, which reflect the highest success rate attained by other users with similar conditions or goals.

According to embodiments, when a user inputs the user's current condition (e.g., weight, height, body fat percentage, etc.) and goal, the mobile healthcare service device 100 may provide individual or team-based competitions based on the rate of goal attainment or health log recording among individuals sharing similar conditions and goals with the user. At this point, the mobile healthcare service apparatus 100 may select competing groups for a user group based on users from other groups with similar conditions and goals, considering factors such as the current status of the user group's goal or conditions and the status of other user groups, aiming for the highest rate of goal attainment or record among these groups.

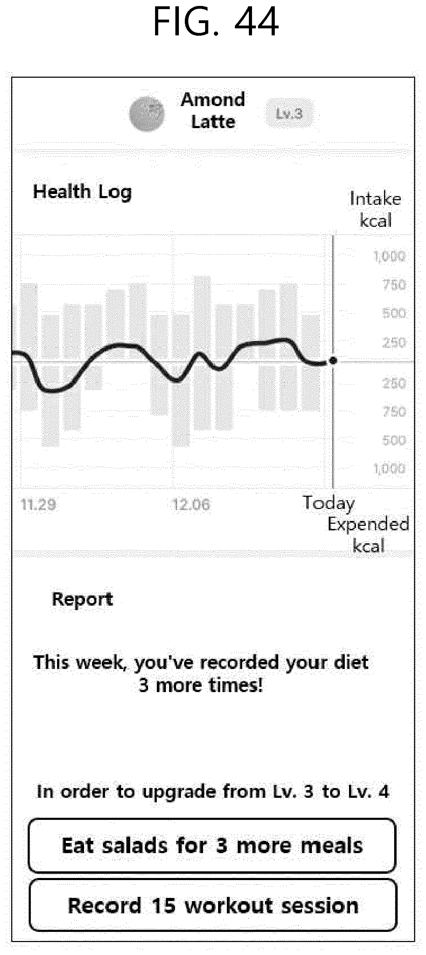

FIG.44 is a diagram showing an example of mentoring and goal attainment mission guide operation by a mobile healthcare service apparatus according to an embodiment of the present disclosure.

As shown in FIG. 44, the mobile healthcare service apparatus 100 according to an embodiment of the present disclosure may provide a chat window for a user to receive mentoring from a mentor the user wish to connect with. The mobile healthcare service apparatus 100 shares the past mentor's health log (e.g., dietary record, exercise record, sleep record, etc.) of the mentor's efforts to attain the goal with the mentee, and provides mentoring to the user on diet, exercise, sleep, etc. can be provided. In addition, the mobile healthcare service apparatus 100 may provide users with step-by-step missions including optimal guidelines (such as dietary management methods, exercise routines, etc.) based on health logs (like dietary records, workout logs, etc.) of users who have endeavored to attain a specific goal (for instance, weight loss, muscle gain, etc.) under particular conditions (for instance, weight, height, body fat percentage, etc.). For example, the mobile healthcare service apparatus 100 may offer mentoring on this week's dietary records or suggest health log actions necessary for an upgrade. In addition, the mobile healthcare service apparatus 100 may operate and provide a diet authentication room for a predetermined period of time for real-time communication.

According to related embodiments, when a user's current conditions (e.g., weight, height, body fat percentage, etc.) and goal is received, the mobile healthcare service apparatus 100 may motivate the user through mentoring by setting another user who has similar conditions with the user and attained a goal similar to the user's goal as a mentor. The mentoring may include advices and discussions on how to attain the goal. In addition, the mobile healthcare service apparatus 100 may provide a goal attainment guide based on the health logs of the mentor who has attained the goal. The mobile healthcare service apparatus 100 may selectively provide guidance prioritized based on the effectiveness of mentoring derived from a mentor's attainments or health logs, considering mentors who are other users with similar conditions and goals as the user.

According to embodiments, when the user's current conditions (e.g., weight, height, body fat percentage, etc.) and goal is received, the mobile healthcare service apparatus 100 may charge the user to access health logs of other users with similar conditions who have attained similar goals. When the user checks the health logs of other users who have attained similar goals and have similar conditions, the mobile healthcare service apparatus 100 may charge varying fees based on the detailed content of health logs or the achievement rate of health logs by categorizing health log grades accordingly.

According to embodiments, when the user's current conditions (e.g., weight, height, body fat percentage, etc.) and goal is received, the mobile healthcare service apparatus 100 may enable the user to view real-time health logs of a nearby user based on similar conditions and goals, and provide a chat window for communication with the nearby user. At this point, the mobile healthcare service apparatus 100 may display real-time health logs of the nearby user based on the current user's location, while also selectively showing and enabling communication with the nearby user whose health logs most closely align with the user's current needs.

According to embodiments, the mobile healthcare service apparatus 100 may expand mobile healthcare services with a doppelganger search. For example, the mobile healthcare service apparatus 100 may recommend local restaurants favored by other users with similar food preferences to the user. Alternatively, the mobile healthcare service apparatus 100 may recommend restaurants at a dining location with suitable menu items based on past consumed foods or preferences stored in the meal partner's mobile healthcare app (e.g., friends, colleagues, clients) for meal arrangements. Alternatively, the mobile healthcare service apparatus 100 may encourage the upload of food data or information by users in a crowdsourcing manner. For example, the mobile healthcare service apparatus 100 may provide rewards when a recommender directly inputs preferences, dislikes, or dietary recommendations for specific dishes at a recommended restaurant, based on previous favorable experiences or dietary management needs. Here, restaurants and menu items may be retrieved from various regional restaurant recommending blogs on the web. Alternatively, the mobile healthcare service apparatus 100 may share dietary improvement patterns observed in individuals successful in dieting or dietary management, who have similar food preferences with the user, and may also reward these individuals for sharing the dietary improvement patterns. Alternatively, the mobile healthcare service apparatus 100 may visualize various diet information of users with similar taste categories.

Meanwhile, according to an embodiment of the disclosure, the various embodiments described above may be implemented as software including instructions stored in machine-readable storage media, which can be read by machines (e.g., computers). Here, the machines refer to apparatuses that call instructions stored in a storage medium, and can operate according to the called instructions, and the apparatuses may include the electronic apparatus according to the aforementioned embodiments (e.g., an electronic apparatus A). In case an instruction is executed by a processor, the processor may perform a function corresponding to the instruction by itself, or by using other components under its control. An instruction may include a code that is generated or executed by a compiler or an interpreter. A storage medium that is readable by machines may be provided in the form of a non-transitory storage medium. Here, the term 'non-transitory' only means that a storage medium does not include signals, and is tangible, but does not indicate whether data is stored in the storage medium semi-permanently or temporarily.

In addition, according to an embodiment of the disclosure, methods according to the aforementioned various embodiments of the disclosure may be provided while being included in a computer program product. A computer program product refers to a product, and it can be traded between a seller and a buyer. A computer program product can be distributed on-line in the form of a storage medium that is readable by machines (e.g., a compact disc read only memory (CD-ROM)), or through an application store (e.g., Play Store^{™}). In the case of on-line distribution, at least a portion of a computer program product may be stored in a storage medium, such as the server of the manufacturer, the server of the application store, and the memory of the relay server at least temporarily, or may be generated temporarily.

Various embodiments described herein may be implemented in a computer-readable medium or similar medium using, for example, software, hardware, or any combination thereof. In some cases, the embodiments described herein may be implemented in a processor itself. For software implementation, procedures or functions described herein may be implemented by separate software modules. Each software module may perform one or more functions or operations described herein.

Meanwhile, computer instructions for performing processing operations of devices according to the various embodiments described above may be stored in a non-transitory computer-readable medium. Computer instructions stored in such a non-transitory computer-readable medium, when executed by a processor of a specific device, cause the specific device to perform processing operations in the device according to the various embodiments described above. A non-transitory computer-readable medium refers to a medium that stores data semi-permanently and can be read by a device, rather than a medium that stores data for a short period of time, such as registers, caches, and memories. Specific examples of non-transitory computer-readable media may include CD, DVD, hard disk, Blu-ray disk, USB, memory card, ROM, etc.

In addition, each of the components according to the aforementioned various embodiments (e.g., a module or a program) may include a singular object or a plurality of objects. In addition, among the aforementioned corresponding sub components, some sub components may be omitted, or other sub components may be further included in the various embodiments. Generally or additionally, some components (e.g., a module or a program) may be integrated as an object, and perform the functions that were performed by each of the components before integration identically or in a similar manner. A module, a program, or operations performed by other components according to the various embodiments may be executed sequentially, in parallel, repetitively, or heuristically. Or, at least some of the operations may be executed in a different order or omitted, or other operations may be added.

While the disclosure has been shown and described with reference to various embodiments thereof, it will be understood by those skilled in the art that various changes in form and details may be made therein without departing from the spirit and scope of the disclosure as defined by the appended claims and their equivalents.

## Claims

1. A mobile healthcare service method performed by a mobile healthcare service apparatus, the method comprising:
when any food is detected on a photoshoot screen upon execution of a camera by a user, displaying a detected food area;
when the user captures a photo of the photoshoot screen displaying the food area, cropping the food area from the captured photo;
when a consumed food is selected by the user in the cropped food area, recording the selected consumed food as dietary data; and
providing a mobile healthcare service using diet management by comparing the dietary data with a preset diet management goal.

2. The method of claim 1, wherein in displaying the food area, a distinct food area for each type of food located in the photoshoot screen is displayed.

3. The method of claim 1, wherein in recording the selected consumed food as the dietary data, the dietary data comprises metadata included in the selected consumed food and the captured photo.

4. The method of claim 1, further comprising:
generating an avatar in a virtual space based on the user's body information, creating virtual clothing, dressing the avatar with the generated virtual clothing, and adjusting a clothing fit of the avatar wearing the virtual clothing based on the recorded dietary data and the preset dietary management goal.

5. The method of claim 1, wherein in recording the selected consumed food as the dietary data, a quantity of the selected consumed food is calculated using restaurant menu data corresponding to the user's location information, and the calculated quantity is recorded as dietary data.

6. The method of claim 1, wherein in providing the mobile healthcare service, a recommended meal order for consuming the detected food is provided to the user based on the preset diet management goal.

7. The method of claim 1, wherein in providing the mobile healthcare service, a food intake warning is issued for any detected food that doesn't align with the preset dietary management goal.

8. The method of claim 1, further comprising:
searching for a doppelgänger user exceeding a preset similarity threshold with the recorded dietary data and the preset dietary management goal, and providing dietary data and diet preference information of the found doppelgänger user.

9. The method of claim 8, further comprising:
providing at least one recommendation information suitable for the user based on the found doppelganger user's dietary data and preference information.

10. A mobile healthcare service apparatus using diet management, the apparatus comprising:
a camera;
a display;
a memory configured to store one or more programs; and
a processor configured to execute the one or more stored programs,
wherein the processor is further configured to:
when any food is detected on a photoshoot screen of the display upon execution of the camera by a user, display a detected food area,
when the user captures a photo of the photoshoot screen showing the food area, crop the food area from the captured photo,
when a consumed food is selected by the user in the cropped food area, recording the selected consumed food as dietary data, and
provide a mobile healthcare service using diet management by comparing the recorded dietary data with a preset diet management goal.

11. The apparatus of claim 10, wherein the processor is further configured to display a distinct food areas for each type of food located in the photoshoot screen of the display.

12. The apparatus of claim 10, wherein the processor is further configured to record dietary data that comprises metadata included in the selected consumed food and the captured photo.

13. The apparatus of claim 10, wherein the processor is further configured to generate an avatar in a virtual space based on the user's body information, create virtual clothing, dress the avatar with the generated virtual clothing, and adjust a clothing fit of the avatar dressed with the virtual clothing based on the recorded dietary data and the preset dietary management goal.

14. The apparatus of claim 10, wherein the processor is further configured to calculate a quantity of the selected consumed food using restaurant menu data corresponding to the user's location information and record the calculated quantity as dietary data.

15. The apparatus of claim 10, wherein the processor is further configured to provide the user with a recommended meal order for consuming the detected food based on the preset diet management goal.
